(19) Europäisches Patentamt
European Patent Office
Office européen des brevets

(11) Publication number: **0 059 090**
**B1**

(12) # EUROPEAN PATENT SPECIFICATION

(45) Date of publication of patent specification: **29.01.86**

(21) Application number: **82300861.0**

(22) Date of filing: **19.02.82**

(51) Int. Cl.[4]: **C 07 D 233/64,**
C 07 D 233/66,
C 07 D 263/32,
C 07 D 263/34,
C 07 D 277/24,
C 07 D 277/32, C 07 D 513/04
// A61K31/415, A61K31/42,
A61K31/425

(54) **3,5-Di-tert-butyl-4-hydroxyphenyl-substituted heterocyclic compounds.**

(30) Priority: **19.02.81 JP 23515/81**
**21.04.81 JP 59990/81**
**02.10.81 JP 157010/81**

(43) Date of publication of application:
**01.09.82 Bulletin 82/35**

(45) Publication of the grant of the patent:
**29.01.86 Bulletin 86/05**

(84) Designated Contracting States:
**AT BE CH DE FR GB IT LI LU NL SE**

(56) References cited:
**EP-A-0 000 353**
**EP-A-0 013 560**
**EP-A-0 013 561**
**AU-A- 475 623**
**US-A-3 578 671**
**US-A-4 072 689**
**US-A-4 104 400**

**Burgers Medicinal Chemistry, 4th Ed, Pt. III**
**Wolff, John Wiley 1981 pps 1251-1252, 1270**

(73) Proprietor: **YAMANOUCHI PHARMACEUTICAL CO. LTD.**
**No. 5-1 Nihonbashi-Honcho, 2-chome Chuo-ku**
**Tokyo (JP)**

(72) Inventor: **Kubo, Kazuo**
**No. 21-4, Negishi 2-chome**
**Urawa-shi Saitama (JP)**
Inventor: **Isomura, Yasuo**
**No. 12-11-919, Nishioku 4-chome Arakawa-ku**
**Tokyo (JP)**
Inventor: **Sakamoto, Shuichi**
**No. 2-1258-201, Ogawacho**
**Kodaira-shi Tokyo (JP)**
Inventor: **Homma, Hiroshige**
**No. 139-2, Kamikomachi**
**Omiya-shi Saitama (JP)**

(74) Representative: **Geering, Keith Edwin et al**
**REDDIE & GROSE 16 Theobalds Road**
**London WC1X 8PL (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

**EP 0 059 090 B1**

**Description**

This invention relates to 3,5-di-tert-butyl-4-hydroxyphenyl-substituted heterocyclic compounds, their preparation, and their pharmaceutical use.

According to the invention there are provided the 3,5-di-tert-butyl-4-hydroxyphenyl-substituted heterocyclic compounds of formula I, and their salts:

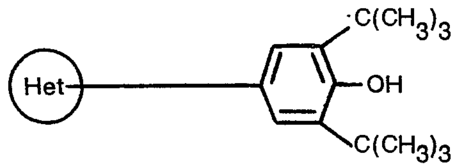

I

wherein

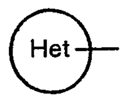

is a heterocyclic group of the formula

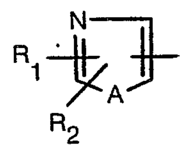

wherein $R_1$ and $R_2$ are the same or different and selected from hydrogen, $C_1$ to $C_7$ alkyl groups, aryl-substituted $C_1$ to $C_7$ alkyl groups, aryl groups, $C_1$ to $C_7$ alkoxy-substituted aryl groups, and the groups —O—Z,

$$-\underset{(O)_n}{S}-Z,$$

and —NH—Z (wherein Z represents hydrogen, a $C_1$ to $C_7$ alkyl group, a carboxy $C_1$ to $C_7$ alkyl group, a ($C_1$ to $C_7$ alkoxy) carbonyl $C_1$ to $C_7$ alkyl group, an aryl-substituted $C_1$ to $C_7$ alkyl group, or an aryl group and n is 0, 1, or 2); A is oxygen, sulfur, or

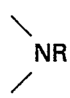

where R is hydrogen or $C_1$ to $C_7$ alkyl or 3,5-di-tert-butyl-4-hydroxyphenacyl

In the following description, the term "lower" means a straight or branched carbon chain of 1—7 carbon atoms. For example, "lower alkyl" includes methyl, ethyl, propyl, isopropyl, butyl, isobutyl, tert-butyl, heptyl, and hexyl; "lower aralkyl" which means aryl-substituted lower alkyl, includes benzyl and phenethyl; "lower alkoxy" includes methoxy, ethoxy, propoxy, isopropoxy, butoxy, and hexyloxy. Furthermore, "aryl" includes phenyl and naphthyl etc.

The salts of this invention include the pharmaceutically acceptable acid addition salts, for example inorganic acids such as hydrochloric, hydrobromic, sulfuric, nitric and phosphoric acids.

Compounds of this invention have variously shown anti-inflammatory, anti-pyretic, analgesic, antiarthritic and immuno-regulatory activities.

Compounds of this invention show therapeutic and prophylactic effects on adjuvant-induced arthritis which is considered to be an animal model of human rheumatism, have analgesic and anti-inflammatory activities and, as a biochemical activity, inhibit prostaglandin formation; they are thus considered to be useful for the therapy and prophylaxis of human rheumatic disease. Moreover, compounds of this invention suppress Coomb's type III (Arthus reaction) and type IV (delayed type hypersensitivity) allergic reactions, as well as having a lipoxigenase suppressing activity, and acting as radical scavengers, unlike conventional (acidic) nonsteroidal anti-inflammatory antirheumatics as represented by indomethacin and diclofenac. The compounds of this invention thus seem to act as antirheumatics by a new mechanism.

One feature of the compounds of this invention is that they are nonacidic nonsteroidal anti-inflammatory agents, as compared to the conventional, acidic nonsteroidal anti-inflammatory agents such as indomethacin and diclofenac. Another feature is that the heterocyclic ring is directly substituted by a 3,5-di-tert-butyl-4-hydroxyphenyl group. A third feature is that the basic substituted heterocyclic ring itself is

selected from specific heterocyclic rings as defined above, and can be an imidazole ring, a thiazole ring or oxazole ring.

As heterocyclic compounds the heterocyclic ring of which is directly substituted by a 3,5-di-tert-butyl-4-hydroxyphenyl group, there are known, for example, 2-(3,5-di-tert-butyl-4-hydroxyphenyl)benzoxazole compounds and 2-(3,5-di-tert-butyl-4-hydroxyphenyl)benzothiazole compounds (West German Offenlegungsschrift 2,008,414); 2-(3,5-di-tert-butyl-4-hydroxyphenyl)-1,3-diphenylimidazolidine compounds (Belgian Patent No. 807,140); and 2-[2-(3,5-di-tert-butyl-4-hydroxyphenyl)-5-ethoxy-4-thiazolyl]acetic acid (Japanese Pat. Appln. Laid Open No. 7669/'78). The benzoxazole compounds and the benzothiazole compounds are used as antioxidants and the imidazolidine compounds are used as intermediates for plant protecting agents and dyes. The latter acetic acid compound is suggested for use as an anti-thrombotic agent, a hypolipaemic agent, and an anti-inflammatory agent, but there is no disclosure about its practical pharmacological effect as an anti-inflammatory agent and it is in any case an acidic non-steroidal anti-inflammatory agent and hence chemically different from the non-acidic compounds of this invention.

Throughout the structural formulae in this specification, the same symbols retain the same meanings unless otherwise specified.

One group of compounds of this invention are of formula Ia:

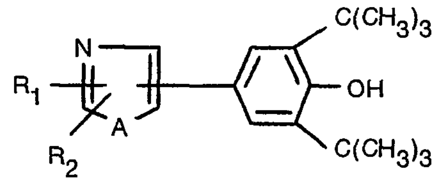

Ia

Particularly preferred compounds of formula Ia are as follows:
4-(3,5-di-tert-butyl-4-hydroxyphenyl)-5-methyl-2-oxo-4-imidazoline,
4-(3,5-di-tert-butyl-4-hydroxyphenyl)-5-ethyl-2-oxo-4-imidazoline,
4-(3,5-di-tert-butyl-4-hydroxypheny)-5-methyl-2-methylthioimidazole,
4-(3,5-di-tert-butyl-4-hydroxyphenyl)-5-methylimidazole,
4-(3,5-di-tert-butyl-4-hydroxyphenyl)-2-mercaptothiazole,
4-(3,5-di-tert-butyl-4-hydroxyphenyl)-2-methylthiothiazole,
4-(3,5-di-tert-butyl-4-hydroxyphenyl)-5-ethylimidazole, and
4-(3,5-di-tert-butyl-4-hydroxyphenyl)-5-ethyl-2-methylthioimidazole.

Some compounds of this invention occur as prototropic isomers. For example, in the compounds of formula Ia wherein $R_1$ is —SH or —OH there are the following prototropic isomers:

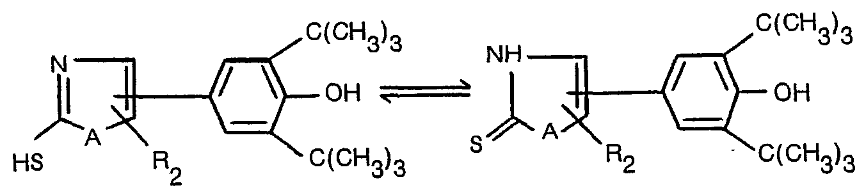

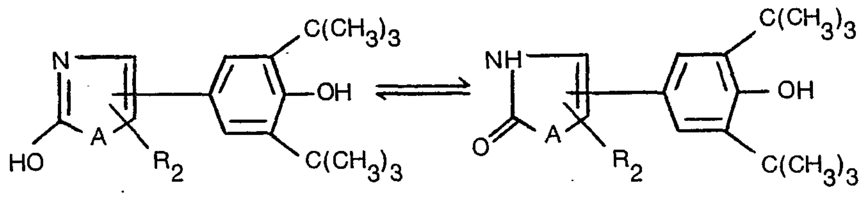

Additionally, tautomerism can occur in the imidazole derivatives having no N-substituent, e.g.

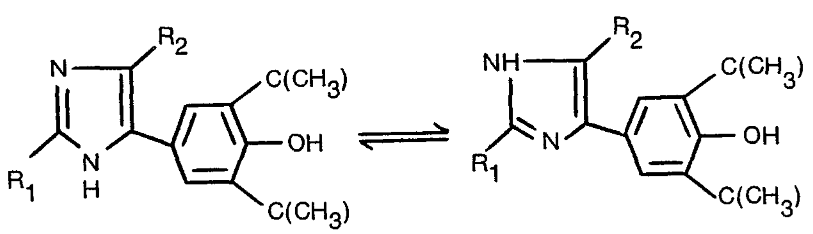

Where a single name or structure is quoted herein for a compound according to the invention, any such tautomeric form in which the compound occurs is included.

The compounds of formula Ia can be shown by formula Ia—1

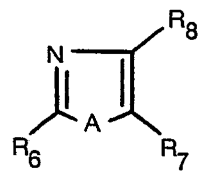

Ia—1

wherein one of $R_6$, $R_7$, and $R_8$ is the 3,5-di-tert-butyl-4-hydroxyphenyl group

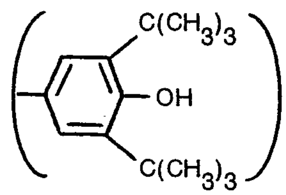

and the others have the same significance as $R_1$ and $R_2$ above; these compounds can be prepared by the following methods:

Ai). First production method:

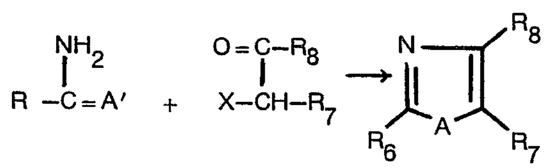

IIa IIIa Ia—1

wherein A' is oxygen, sulfur, an imino group or a lower alkylimino group; and X is halogen.

Thus a compound Ia—1 can be prepared by heating a compound IIa (e.g. a urea, thiourea, amidine, amide, thioamide, or amidothiocarbonic acid derivative) with a corresponding amount of α-halocarbonyl compound IIIa alone or in a suitable solvent such as methanol, ethanol, toluene, dimethylformamide, acetone, chloroform, methyl ethyl ketone, ethyl acetate, methyl CELLOSOLVE (TM), ethyl CELLOSOLVE (TM), diglyme or acetonitrile. The reaction temperature and time depend upon starting materials and solvent used. The compound IIa may be used for the reaction in salt form, such as a hydrochloride of an amidine derivative or ammonium amidodithiocarbonate.

When cyanourea ($NH_2CONHCN$) is reacted with an α-halocarbonyl compound IIIa and the product hydrolyzed, a compound of formula

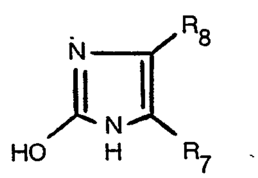

can be produced.

Aii) Second production method:

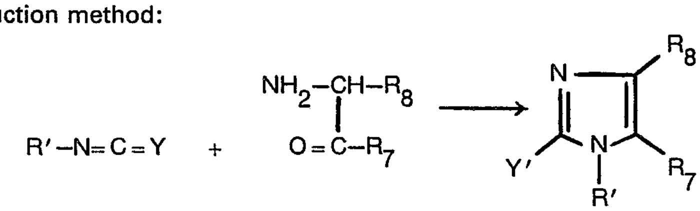

IVa Va Ia—2

wherein R' is hydrogen or a lower alkyl group; Y' is a hydroxyl group or a mercapto group; and Y is oxygen or sulfur.

Thus a compound Ia—2 can be prepared by reacting a compound IVa (i.e. isocyanic acid, isothiocyanic acid, or a lower alkyl-substituted derivative thereof) or an alkali metal salt thereof with an α-aminocarbonyl compound Va or a salt thereof. When using an alkali metal salt of isocyanic acid or isothiocyanic acid, the reaction is suitably performed in a solvent such as alcohol or aqueous alcohol, with the addition of an acid such as hydrochloric acid, at room temperature or under heating. When heating is used, the reaction may be performed under reflux.

When using a lower alkyl-substituted derivative of isocyanic acid or isothiocyanic acid, the reaction is best performed in a basic solvent such as pyridine, at room temperature or under heating.

A compound of formula

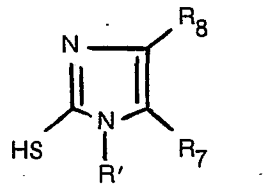

can be prepared by reacting a compound

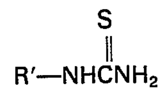

with a compound

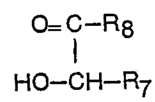

Aiii). Other production methods:

(1) Oxidation

Ia–3 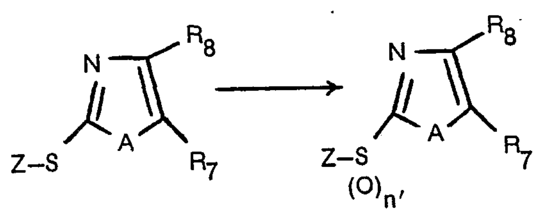 Ia–4

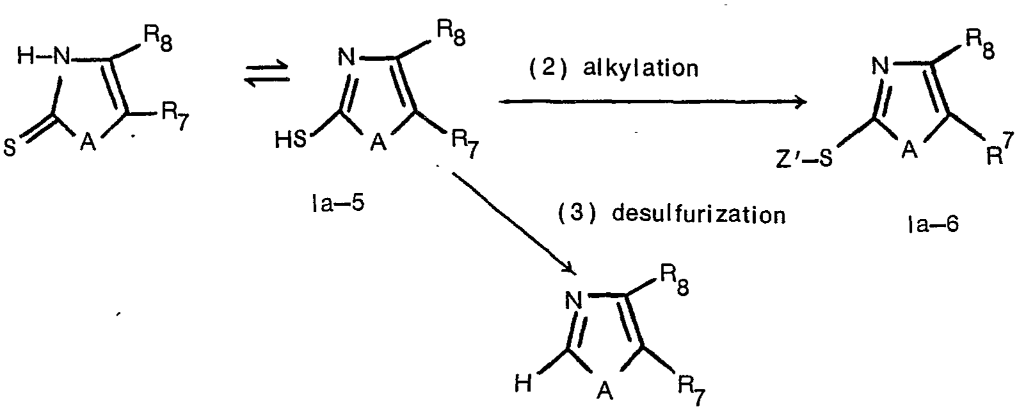


Ia–5

Ia–6

Ia–7

wherein n′ is 1 or 2; and Z′ is a lower alkyl, carboxy lower alkyl, lower alkoxycarbonyl lower alkyl, or lower aralkyl group.

In the oxidation method (1) an S-oxide compound Ia—4 can be prepared by reacting the corresponding thio compound Ia—3 with oxidizing agent in a solvent such as acetic acid, chloroform or 1,2-dimethoxyethane in conventional manner. Suitable oxidizing agents are 10—40% hydrogen peroxide, perbenzoic acid and m-chloroperbenzoic acid. By suitably selecting the reaction conditions such as reaction time and temperature and the amount of oxidizing agent, the monoxide (n' = 1) or dioxide (n' = 2) compound can be obtained as desired.

In the alkylation method (2) an alkylthio compound Ia—6 can be produced by alkylating a mercapto (or thioxo) compound Ia—5 with alkylating agent in a solvent such as methanol, acetone or dimethylformamide in conventional manner. Suitable alkylating agents for the reaction include alkyl halides or aralkyl halides such as methyl iodide, ethyl bromide, α-bromopropionic acid, α-bromoacetic acid, ethyl α-bromopropionate, benzyl bromide and phenethyl iodide, and dialkyl sulfates such as dimethyl sulfate and diethyl sulfate.

In the desulfurization reaction (3) a compound Ia—7 can be produced by desulfurizing the corresponding mercapto (or thioxo) compound Ia—5 in conventional manner, for example by treatment with a Raney nickel catalyst.

The compounds of this invention can be isolated and purified by conventional chemical operations such as concentration, recrystallization, and column chromatography.

The results of tests showing the pharmacological effects of compounds of this invention are as follows.

Effect on adjuvant-induced arthritis in rats:

Method: Male Sprague Dawley rats aged 7 weeks were used. Drugs were evaluated by two methods as follows. All test drugs were suspended in water with 0.5% methylcellulose (0.5% MC) and administered orally (P.O.) once a day.

(I) Therapeutic effect of drugs: arthritis was induced by a single injection of 0.1 ml of sterile suspension of *Mycobacterium butyricum* (6 mg/ml) in liquid paraffin into the tail skin of rats (day 0). After about 2 weeks, arthritic rats were selected and allocated into groups. Drugs were given daily thereafter for about 10 days. Foot thickness was measured with a dial thickness gauge both on the day of initial dosing and on the day after final dosing, and the change of foot thickness (ΔFT $10^{-2}$ mm) was calculated as the difference between these two values. The results are shown in Table I. (N = number of animals). (a) Prophylactic effect of drugs: arthritis was induced by single subplantar injections of 0.05 ml of the suspension into the left hind paws of rats. Drugs were given daily for 21 days starting from the day (day 0) of injection of the suspension. The thicknesses of both rear feet, injected foot (left foot, $FT_L$) and uninjected foot (right foot, $FT_R$) were measured with a dial thickness gauge on days 0 and 21. The percent inhibition (I%) was calculated from the difference in increased foot thickness between the control and drug-treated groups. The results are shown in Table II. (N = number of animals).

TABLE 1
(Therapeutic effect)

| Drug | Dose (mg/kg/day P.O. | N | Day 16 – Day 27 ΔFT ($\times 10^{-2}$ mm) |
|---|---|---|---|
| 0.5% MC | — | 3 | 233 ± 59 |
| Indomethacin | 2 | 3 | −173 ± 62*** |
| 4-(3,5-di-tert-butyl-4-hydroxyphenyl)-5-methyl-2-oxo-4-imidazoline | 25 | 3 | −216 ± 30*** |

| Drug | Dose (mg/kg/day P.O. | N | Day 16 – Day 25 ΔFT ($\times 10^{-2}$ mm) |
|---|---|---|---|
| 0.5% MC | — | 3 | 97 ± 91 |
| Indomethacin | 2 | 3 | −216 ± 30* |
| 4-(3,5-di-tert-butyl-4-hydroxyphenyl)-5-methyl-2-methylthioimidazole | 25 | 3 | −153 ± 56* |
| 4-(3,5-di-tert-butyl-4-hydroxyphenyl)-5-methylimidazole | 25 | 3 | −122 ± 46* |

| Drug | Dose (mg/kg/day P.O. | N | Day 16 – Day 28 ΔFT ($10^{-2}$ mm) |
|---|---|---|---|
| 0.5% MC | — | 6 | 148 ± 37 |
| Indomethacin | 2 | 6 | −204 ± 33*** |
| 4-(3,5-di-tert-butyl-4-hydroxyphenyl)-2-mercaptothiazole | 25 | 3 | −57 ± 26*** |
| 4-(3,5-di-tert-butyl-4-hydroxyphenyl)-2-methylthiazole | 25 | 3 | −166 ± 59*** |
| 4-(3,5-di-tert-butyl-4-hydroxyphenyl)-5-ethyl-2-oxo-4-imidazole | 25 | 3 | −247 ± 49*** |
| 4-(3,5-di-tert-butyl-4-hydroxyphenyl)-5-ethylimidazole | 25 | 3 | −103 ± 71** |

| Drug | Dose (mg/kg/day P.O. | N | Day 15 – Day 28 ΔFT ($10^{-2}$ mm) |
|---|---|---|---|
| 0.5% MC | — | 3 | 96 ± 162 |
| Indomethacin | 2 | 3 | −161 ± 137* |
| 4-(3,5-di-tert-butyl-4-hydroxyphenyl)-5-ethyl-2-methylthioimidazole | 25 | 3 | −180 ± 241* |

| Drug | Dose (mg/kg/day P.O. | N | Day 15 – Day 29 ΔFT (I%) ($10^{-2}$ mm) |
|---|---|---|---|
| 0.5% MC | — | 6 | 280 ± 54 |
| Indomethacin | 1 | 6 | −243 ± 16*** (69.1) |
| | 2 | 6 | −260 ± 24*** (73.7) |
| 4-(3,5-di-tert-butyl-4-hydroxyphenyl)-5-methyl-2-oxo-4-imidazoline | 6.25 | 6 | 67 ± 48** (30.8) |
| do. | 12.5 | 6 | −114 ± 33*** (53.9) |
| do. | 25 | 6 | −194 ± 36*** (66.7) |
| 4-(3,5-di-tert-butyl-4-hydroxyphenyl)-5-ethyl-2-oxo-4-imidazoline | 6.25 | 6 | −19 ± 39*** (41.3) |
| do. | 12.5 | 6 | −176 ± 27*** (63.1) |
| do. | 25 | 6 | −127 ± 38*** (54.0) |

Significant difference from control (t-test)

* P<0.05 ** P<0.01 *** P<0.001

TABLE II
(Prophylactic effect)

| Drug | Dose (mg/kg/day P.O.) | N | Day 21 $FT_L$ I% | Day 21 $FT_R$ I% |
|---|---|---|---|---|
| Indomethacin | 1 | 8 | 64.6 | 81.8 |
| do. | 2 | 8 | 59.5 | 76.9 |
| 4-(3,5-di-tert-butyl-4-hydroxyphenyl)-5-methyl-2-methylthioimidazole | 25 | 8 | 53.1 | 65.8 |
| 4-(3,5-di-tert-butyl-4-hydroxyphenyl)-5-methylimidazole | 25 | 8 | 68.1 | 81.3 |

| Drug | Dose (mg/kg/day P.O.) | N | Day 21 FT$_L$ I% | FT$_R$ I% |
|---|---|---|---|---|
| Indomethacin | 1 | 8 | 63.6 | 68.1 |
| do. | 2 | 8 | 63.9 | 98.0 |
| 4-(3,5-di-tert-butyl-4-hydroxyphenyl)-5-methyl-2-oxo-4-imidazoline | 6.25 | 8 | 38.0 | 71.7 |
| do. | 12.5 | 8 | 30.3 | 61.6 |
| do. | 25 | 8 | 64.4 | 90.9 |
| 4-(3,5-di-tert-butyl-4-hydroxyphenyl)-5-ethyl-2-oxo-4-imidazoline | 6.25 | 8 | 11.6 | 35.8 |
| do. | 12.5 | 8 | 26.3 | 47.2 |
| do. | 25 | 8 | 60.1 | 70.1 |

The preparation of compounds according to the invention is illustrated by the following Examples:

Example 1

A mixture of 2.4 g of N-(2-phenylethyl)thiourea, 5 g of 4-(2-bromoacetyl)-2,6-di-tert-butylphenol, and 25 ml of absolute ethanol was refluxed for 3 hours and then cooled. The precipitates thus formed were recovered by filtration and recrystallized from ethanol to provide 2.5 g of 4-(3,5-di-tert-butyl-4-hydroxyphenyl)-2-(-α-phenethylamino)thiazole hydrobromide.

Melting point: 228—230°C

Elemental analysis for $C_{25}H_{33}N_2OSBr$:

| | C (%) | H (%) | N (%) |
|---|---|---|---|
| Calculated: | 61.34 | 6.79 | 5.72 |
| Found: | 61.65 | 6.96 | 5.63 |

Example 2

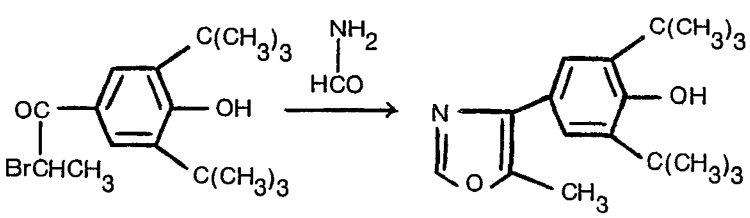


A mixture of 4.5 g of formamide and 2.25 g of 4-(2-bromopropionyl)-2,6-di-tert-butylphenol was heated to 150°C for 1.5 hours. The reaction mixture was poured into water after cooling and then extracted twice, each time with 30 ml of toluene. The extract was dried over anhydrous magnesium sulfate and concentrated under reduced pressure and the residue was recrystallized from aqueous methanol to provide 1.1 g of 4-(3,5-di-tert-butyl-4-hydroxyphenyl)-5-methyloxazole.

Melting point: 105—107°C

Elemental analysis for $C_{18}H_{25}NO_2$:

| | C (%) | H (%) | N (%) |
|---|---|---|---|
| Calculated: | 75.23 | 8.77 | 4.87 |
| Found: | 75.07 | 8.91 | 4.68 |

Example 3

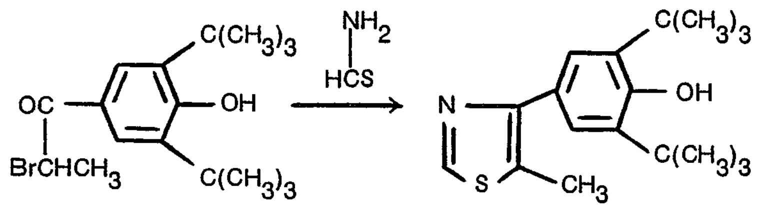

A mixture of 0.7 g of thioformamide, 3.4 g of 4-(2-bromopropionyl)-2,6-di-tert-butylphenyl, and 15 ml of absolute ethanol was heated to 50—60°C for 2—3 hours. After cooling, the reaction mixture poured into a dilute aqueous solution of potassium carbonate. The precipitates thus formed were recovered by filtration and recrystallized from a mixture of cyclohexane and hexane to provide 1.2 g of 4-(3,5-di-tert-butyl-4-hydroxyphenyl)-5-methylthiazole.

Melting point: 128°—130°C

Elemental analysis for $C_{18}H_{25}NOS$:

| | C (%) | H (%) | N (%) |
|---|---|---|---|
| Calculated: | 71.24 | 8.30 | 4.62 |
| Found: | 71.39 | 8.53 | 4.38 |

Example 4

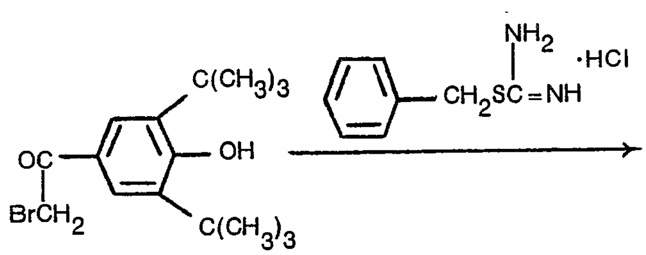

and

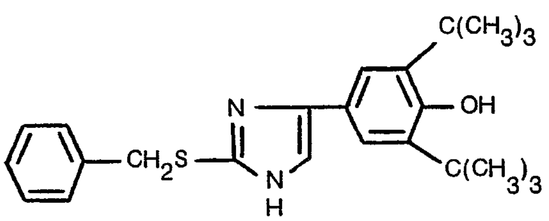

(a)

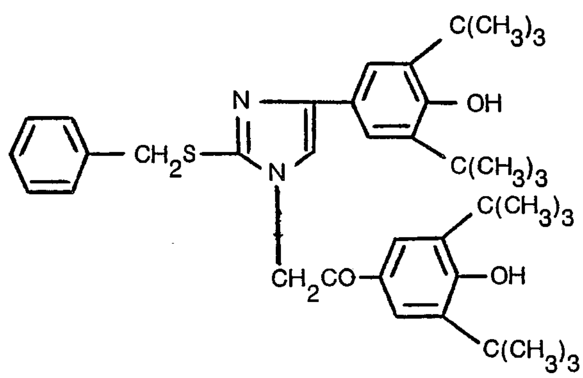

(b)

A mixture of 4 g of benzylthioformamidine hydrochloride, 6.4 g of 4-(2-bromoacetyl)-2,6-di-tert-butylphenol, 20 ml of chloroform, and 48 ml of 84% aqueous ethanol was vigorously stirred and then 6.6 g of sodium hydrogen carbonate was added to the mixture at room temperature. Thereafter, the resultant mixture was refluxed for 3 hours. The reaction mixture thus obtained was poured into water and extracted twice, each time with 100 ml of benzene. The extract was dried and then concentrated under reduced pressure. The residue was subjected to silica gel column chromatography and by eluting with chloroform, two fractions were obtained. Each fraction was concentrated and recrystallized from cyclohexane to provide 1.2 g (a) 2-benzylthio-4-(3,5-di-tert-butyl-4-hydroxyphenyl)imidazole and 0.6 g of (b) 2-benzylthio-1-(3,5-di-tert-butyl-4-hydroxyphenacyl)-4-(3,5-di-tert-butyl-4-hydroxyphenyl)imidazole respectively.

(a) Melting point: 92—94°C
Elemental analysis for $C_{24}H_{30}N_2OS + \frac{1}{2}C_6H_{12}$:

| | C (%) | H (%) | N (%) |
|---|---|---|---|
| Calculated: | 74.21 | 8.25 | 6.41 |
| Found: | 73.83 | 8.43 | 6.36 |

(b) Melting point: 99—102°C
Elemental analysis for $C_{40}H_{52}N_2O_3S$:

| | C (%) | H (%) | N (%) |
|---|---|---|---|
| Calculated: | 74.96 | 8.18 | 4.37 |
| Found: | 74.78 | 8.58 | 4.05 |

## Example 5

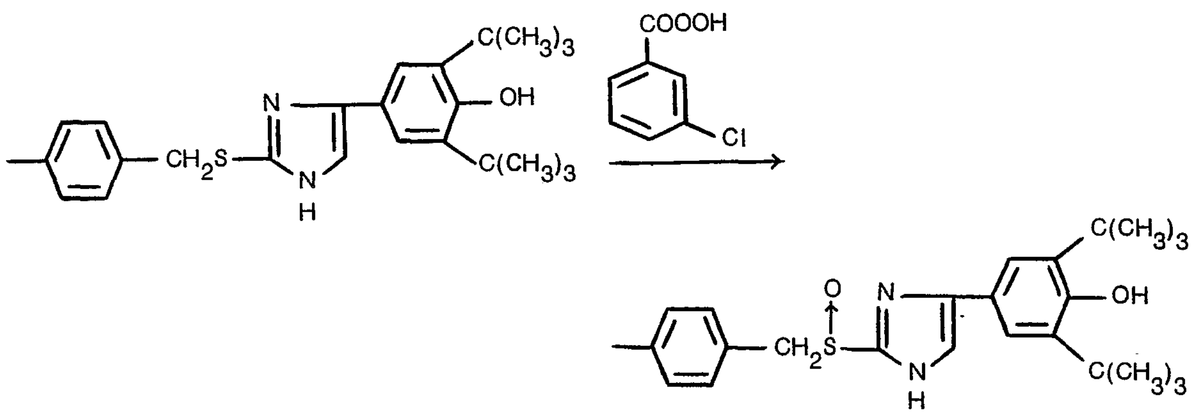

In 10 ml of chloroform was dissolved 0.8 g of 2-benzylthio-4-(3,5-di-tert-butyl-4-hydroxyphenyl)imidazole and then 0.35 g of m-chloroperbenzoic acid was added to the solution at room temperature. After the reaction was over, the reaction mixture was washed with a dilute aqueous alkali solution, dried, and concentrated under reduced pressure. The residue formed was subjected to silica gel chromatography and eluted with chloroform. The fraction thus obtained was concentrated under reduced pressure and the residue was recrystallized from cyclohexane to provide 0.3 g of 2-benzylsulfinyl-4-(3,5-di-tert-butyl-4-hydroxyphenyl)imidazole.

Melting point: 189—191°C
Elemental analysis for $C_{24}H_{30}N_2O_2S.\frac{1}{2}C_6H_{12}$:

| | C (%) | H (%) | N (%) |
|---|---|---|---|
| Calculated: | 71.59 | 7.95 | 6.19 |
| Found: | 71.48 | 8.16 | 6.19 |

## Example 6

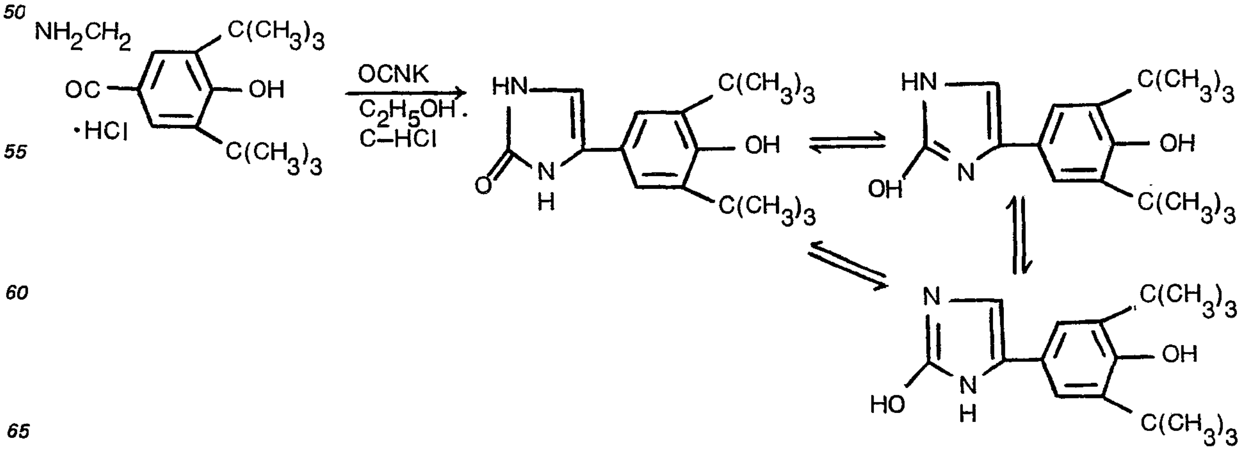

To a mixed solution of 1.2 g of 4-(2-aminoacetyl)-2,6-di-tert-butylphenol hydrochloride, 10 ml of ethanol, and 0.3 ml of concentrated hydrochloric acid was added a solution of 0.64 g of potassium isocyanate in 3 ml of water at room temperature. After stirring the mixture for 2 hours, 0.3 ml of concentrated hydrochloric acid was added thereto and the resultant mixture was refluxed for 2 hours. The reaction mixture thus obtained was poured into water and the precipitates formed were recovered by filtration. The reaction product was then recrystallized from aqueous ethanol to provide 0.5 g of 4-(3,5-di-tert-butyl-4-hydroxyphenyl)-2-oxo-4-imidazoline.

Melting point: above 260°C (decompd.)

Elemental analysis for $C_{17}H_{24}N_2O_2 \cdot \frac{1}{2}C_2H_5OH$:

| | C (%) | H (%) | N (%) |
|---|---|---|---|
| Calculated: | 69.37 | 8.68 | 8.99 |
| Found: | 69.08 | 9.04 | 8.84 |

Example 7

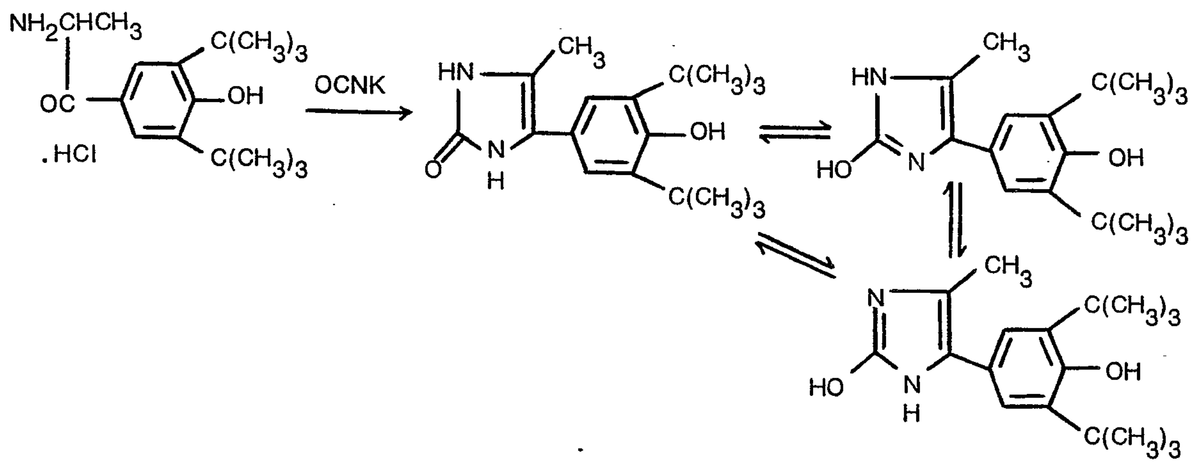


Following the same procedure as in Example 6 using 1.6 g of 4-(2-aminopropionyl)-2,6-di-tert-butyl-4-hydroxyphenol hydrochloride, 20 ml of ethanol, 0.5 ml of concentrated hydrochloric acid, and 0.8 g of potassium isocyanate, a reaction product was obtained and recrystallized from aqueous isopropanol to provide 0.7 g of 4-(3,5-di-tert-butyl-4-hydroxyphenyl)-5-methyl-2-oxo-4-imidazoline.

Melting point: above 270°C (decompd.)

Elemental analysis for $C_{18}H_{26}N_2O_2$:

| | C (%) | H (%) | N (%) |
|---|---|---|---|
| Calculated: | 71.49 | 8.67 | 9.26 |
| Found: | 71.47 | 8.82 | 9.22 |

This product was recrystallized from aqueous ethanol to provide the pure product having a melting point of 279—282°C.

Example 8

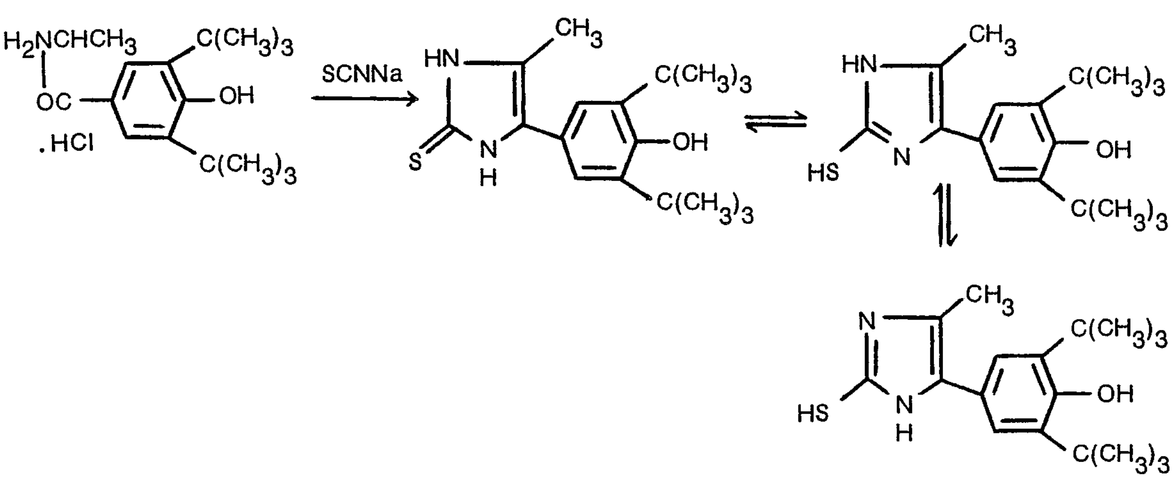

To a mixed solution of 4.3 g of 4-(2-aminopropionyl)-2,6-di-tert-butylphenyl hydrochloride, 50 ml of ethanol, and 1.2 ml of concentrated hydrochloric acid was added a solution of 2.2 g of sodium thiocyanate in 4 ml of water at room temperature. After stirring the mixture for 1.5 hours at room temperature and further refluxing the mixture for 2 hours, the solvent was distilled off under reduced pressure. Then, 30 ml of acetic acid was added to the residue and the mixture was refluxed for 2—3 hours. The reaction mixture thus formed was poured into water and the precipitates formed were recovered by filtration. The product was recrystrallized from isopropanol to provide 3.1 g of 4-(3,5-di-tert-butyl-4-hydroxyphenyl)-5-methyl-2-thioxo-4-imidazoline.

Melting point: 288—290°C

Elemental analysis for $C_{18}H_{26}N_2OS + (CH_3)_2CHOH$:

| | C (%) | H (%) | N (%) |
|---|---|---|---|
| Calculated: | 66.63 | 9.05 | 7.40 |
| Found: | 66.51 | 9.14 | 7.43 |

Example 9

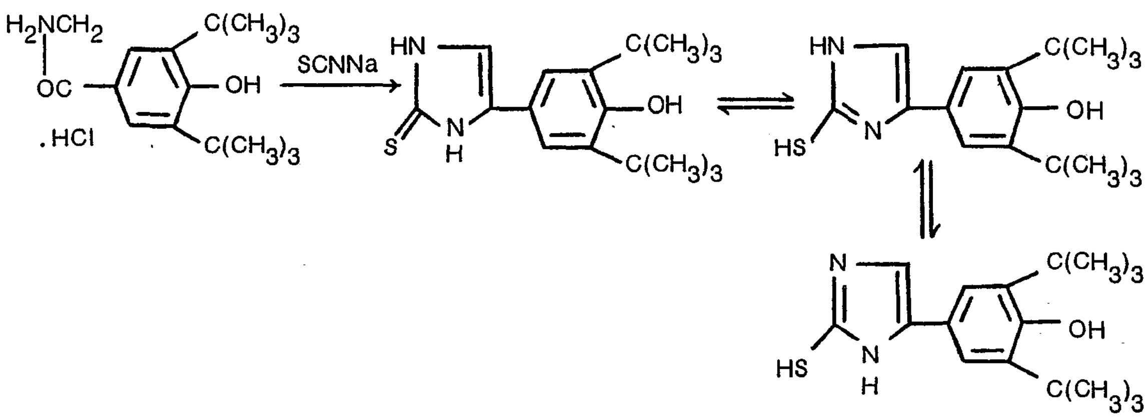


The precipitates obtained by following the same procedure as in Example 8 using 18 g of 4-(2-aminoacetyl)-2,6-di-tert-butylphenol hydrochloride, 150 ml of ethanol, and 5 ml of concentrated hydrochloric acid were subjected to silica gel chromatography and eluted with chloroform. The fraction thus obtained was concentrated under reduced pressure and the residue was recrystallized from benzene-cyclohexane to provide 12 g of 4-(3,5-di-tert-butyl-4-hydroxyphenyl)-2-thioxo-4-imidazoline.

Melting point: 168—170°C

Elemental analysis for $C_{17}H_{24}N_2OS + \frac{1}{2}C_6H_{12}$:

| | C (%) | H (%) | N (%) |
|---|---|---|---|
| Calculated: | 69.27 | 8.06 | 8.08 |
| Found: | 69.33 | 8.46 | 7.76 |

Example 10

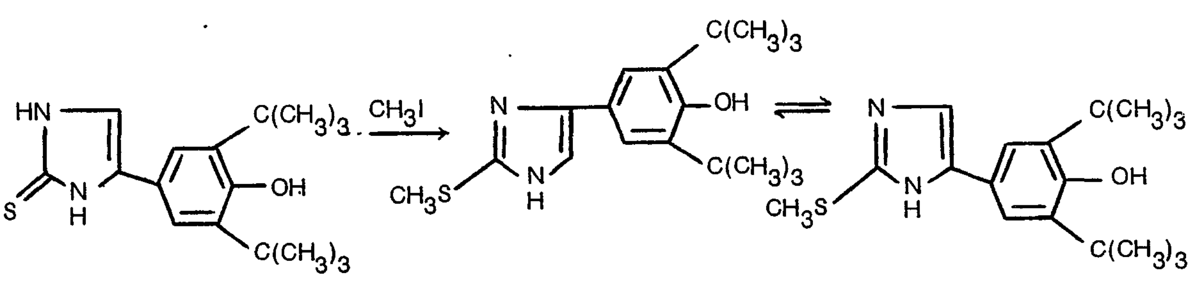


To a mixture of 3.18 g of 4-(3,5-di-tert-butyl-4-hydroxyphenyl)-2-thioxo-4-imidazoline, 50 ml of dry acetone, and 1.3 g of potassium carbonate was added 1.4 g of methyl iodide at room temperature. After stirring the mixture for one hour, the solvent was distilled off. To the residue thus formed was added water, and the precipitates formed were recovered by filtration and dried to provide 2 g of the product. Then 0.8 g of the product was recrystallized from toluene to provide 0.5 g of 4-(3,5-di-tert-butyl-4-hydroxyphenyl)-2-methyl-thioimidazole.

Melting point: 249—251°C

Elemental analysis for $C_{18}H_{26}N_2OS$:

| | C (%) | H (%) | N (%) |
|---|---|---|---|
| Calculated: | 67.89 | 8.23 | 8.80 |
| Found: | 67.62 | 8.36 | 8.70 |

Example 11

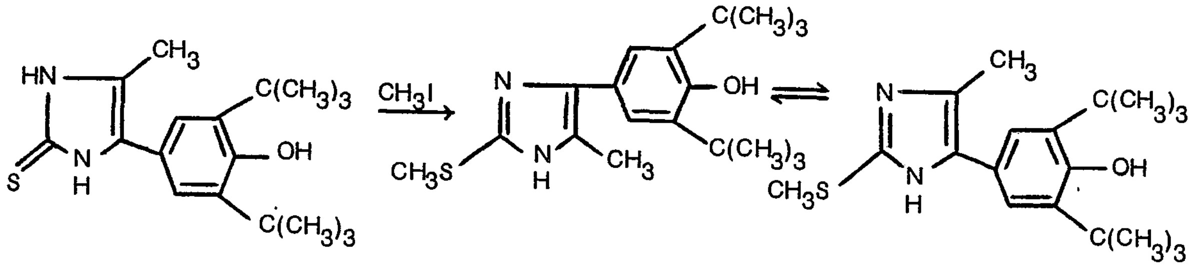


By following the same procedure as in Example 10 using 0.32 g of 4-(3,5-di-tert-butyl-4-hydroxyphenyl)-5-methyl-2-thioxo-4-imidazoline, 10 ml of dry acetone, 0.15 g of potassium carbonate, and 0.15 g of methyl iodide and recrystallizing the product from cyclohexane, 0.1 g of 4-(3,5-di-tert-butyl-4-hydroxyphenyl)-5-methyl-2-methylthioimidazole was obtained.

Melting point: 184—186°C

Elemental analysis for $C_{19}H_{28}N_2OS$:

| | C (%) | H (%) | N (%) |
|---|---|---|---|
| Calculated: | 68.63 | 8.49 | 8.43 |
| Found: | 68.42 | 8.74 | 8.40 |

Example 12

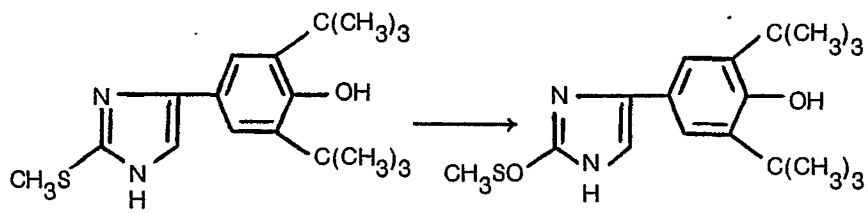

To a solution of 0.32 g of 4-(3,5-di-tert-butyl-4-hydroxyphenyl)-2-methylthioimidazole in 5 ml of chloroform was added 0.18 g of m-chloroperbenzoic acid at room temperature and the mixture was stirred for a day. The reaction mixture was washed with a dilute alkali solution, dried, concentrated under reduced pressure, and the residue thus formed was recrystallized from cyclohexane to provide 0.13 g of 4-(3,5-di-tert-butyl-4-hydroxyphenyl)-2-methylsulfinylimidazole.

Melting point: 106—108°C

Mass spectrum: $M^+$ 334

Nuclear magnetic resonance spectra (in $CDCl_3$): δ (ppm): 1.48 (S; 18H), 3.1 (S; 3H), 5.3 (S; 1H), 7.32 (S; 1H), 7.44 (S; 2H).

Example 13

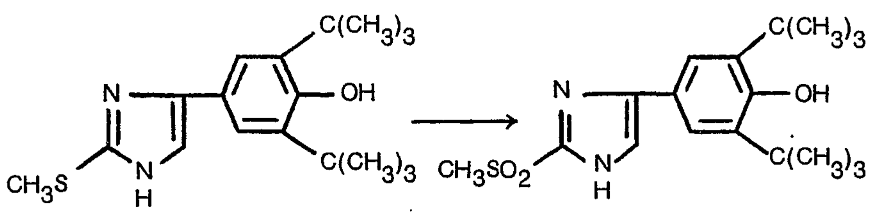

In 10 ml of 1,2-dimethoxyethane was dissolved 0.62 g of 4-(3,5-di-tert-butyl-4-hydroxyphenyl)-2-methylthioimidazole and after adding thereto 0.44 g of m-chloroperbenzoic acid, the mixture was refluxed for 3—4 hours. The reaction mixture was cooled, washed with a dilute alkali solution, dried, and concentrated under reduced pressure. The residue thus formed was recrystallized from cyclohexane to

provide 0.5 g of 4-(3,5-di-tert-butyl-4-hydroxyphenyl)-2-methylsulfonylimidazole.

Melting point: 119—120°C

Mass spectrum: $M^+$ 350

Nuclear magnetic resonance spectra (in $CDCl_3$): δ (ppm): 1.4 (S, 18H), 3.06 (S, 3H), 5.06 (S, 1H), 7.3 (S, 1H), 7.3 (S, 1H), 7.48 (S, 2H).

Example 14

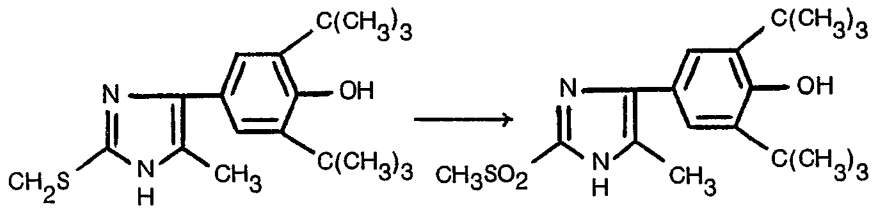

By following the same procedure as in Example 13 using 0.9 g of 4-(3,5-di-tert-butyl-4-hydroxyphenyl)-5-methyl-2-methylthioimidazole, 10 ml of 1,2-dimethoxyethane, and 0.64 g of m-chloroperbenzoic acid and recrystallizing the product from toluene, 0.3 g of 4-(3,5-di-tert-butyl-4-hydroxyphenyl)-5-methyl-2-methylsulfonylimidazole was obtained.

Melting point: 235—237°C

Elemental analysis for $C_{19}H_{28}N_2O_3S$:

| | C (%) | H (%) | N (%) |
|---|---|---|---|
| Calculated: | 62.61 | 7.74 | 7.69 |
| Found: | 62.91 | 8.01 | 7.25 |

Example 15

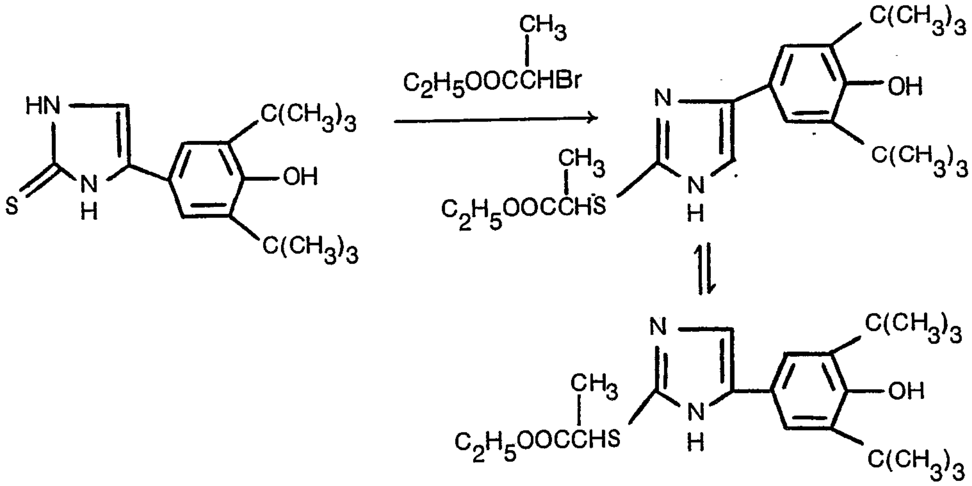


By following the same procedure as in Example 10 using 1.8 g of 4-(3,5-di-tert-butyl-4-hydroxyphenyl)-2-thioxo-4-imidazoline, 30 ml of dry acetone, 0.99 g of potassium carbonate, and 1.3 g of ethyl α-bromopropionate and recrystallizing the product from a mixture of toluene and cyclohexane, 0.95 g of ethyl 2-4-(3,5-di-tert-butyl-4-hydroxyphenyl)-2-imidazolylthio propionate was obtained.

Melting point: 80—82°C

Elemental analysis for $C_{22}H_{32}N_2O_3S$:

| | C (%) | H (%) | N (%) |
|---|---|---|---|
| Calculated: | 65.31 | 7.97 | 6.92 |
| Found: | 65.58 | 8.19 | 6.63 |

Example 16

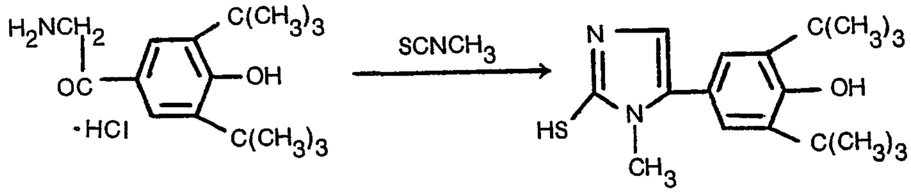


After stirring a mixture of 100 ml of pyridine, 6 g of methyl isothiocyanate, and 3 g of 4-(2-aminoacetyl)-2,6-di-tert-butylphenol hydrochloride at room temperature for 2 hours, the mixture was maintained at a temperature of 80—90°C for 1.5 hours. The reaction mixture was concentrated under reduced pressure and then the residue was extracted with 100 ml of ethyl acetate. The extract was washed with dilute hydrochloric acid, dried, and concentrated under reduced pressure. The residue was re-crystallized from enthanol to provide 0.6 g of 5-(3,5-di-tert-butyl-4-hydroxyphenyl)-2-mercapto-1-methylimidazole.

Melting point: 288—289°C

Nuclear magnetic resonance spectra (in $CDCl_3$): δ(ppm): 1.44 (S, 18H), 3.54 (S, 3H), 5.38 (S, 1H), 6.64 (S, 1H), 7.08 (S, 2H).

Mass spectrum $M^+$ 318

Example 17

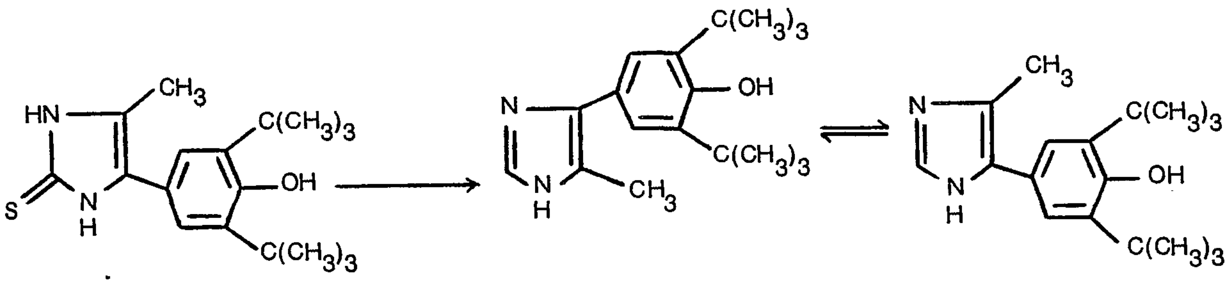

A mixture of 1.5 g of 4-(3,5-di-tert-butyl-4-hydroxyphenyl)-5-methyl-2-thioxo-4-imidazoline, 0.5 g of a Raney nickel catalyst, and 50 ml of absolute ethanol was refluxed for one hour and after filtering the reaction mixture, the filtrate was concentrated under reduced pressure. The residue was recrystallized from a mixture of cyclohexane and n-hexane to provide 1 g of 4-(3,5-di-tert-butyl-4-hydroxyphenyl)-5-methylimidazole.

Melting point: 207—209°C

Elemental analysis for $C_{18}H_{26}N_2O$:

| | C(%) | H(%) | N(%) |
|---|---|---|---|
| Calculated: | 75.48 | 9.15 | 9.78 |
| Found: | 75.31 | 9.40 | 9.53 |

Example 18

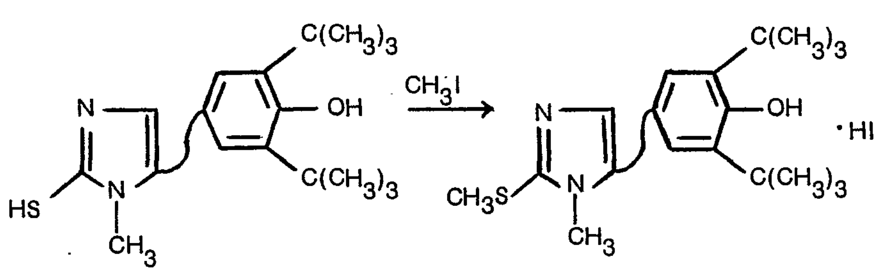


To a solution of 0.6 g of potassium hydroxide dissolved in 10 ml of ethanol was added 1.6 g of 4-(3,5-di-tert-butyl-4-hydroxyphenyl)-2-mercapto-1-methylimidazole at a temperature below 10°C and the mixture was stirred for 20 minutes. Thereafter, 0.7 g of methyl iodide was added to the mixture and after further stirring the mixture for 3 hours at 10°C, the resulting mixture was poured into dilute hydrochloric acid and extracted twice, each time with 50 ml of ethyl acetate. The extract was dried, concentrated under reduced pressure, and then the residue was recrystallized from isopropanol to provide 0.75 g of 5-(3,5-di-tert-butyl-4-hydroxyphenyl)-1-methyl-2-methylthioimidazole hydroiodide.

Melting point: 215—217°C
Elemental analysis for $C_{19}H_{29}N_2OSI$:

| | C(%) | H(%) | N(%) |
|---|---|---|---|
| Calculated: | 49.57 | 6.30 | 6.08 |
| Found: | 49.48 | 6.33 | 5.96 |

Mass spectrum: $M^+$ 332

Example 19

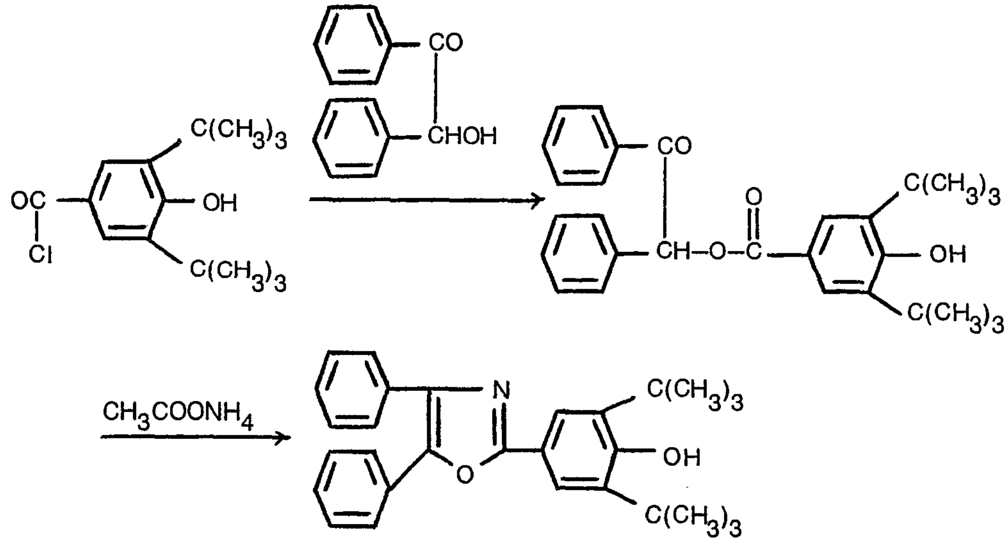


In 10 ml of pyridine was dissolved 2.1 g of benzoin and after adding thereto 2.7 g of 3,5-di-tert-butyl-4-hydroxybenzoyl chloride, the mixture was stirred for 15 hours. The reaction mixture was poured into water and extracted with ethyl acetate. The extract was washed with dilute hydrochloric acid, dried, and then the solvent was distilled off. The crystals thus formed were purified by silica gel column chromatography using chloroform as the eluent to provide 1.7 g of 3,5-di-tert-butyl-4-hydroxybenzoic acid benzoin ester. Then, 1.5 g of the ester was dissolved in 25 ml of acetic acid and after adding 3 g of ammonium acetate to the solution, the mixture was refluxed for one hour. To the reaction mixture was added 20 ml of water and the crystals thus precipitated were recovered by filtration and recrystallized from ethanol to provide 1.0 g of 2-(3,5-di-tert-butyl-4-hydroxyphenyl)-4,5-diphenyloxazole.

Melting point: 185—186°C
Elemental analysis for $C_{29}H_{31}NO_2$:

| | C(%) | H(%) | N(%) |
|---|---|---|---|
| Calculated: | 81.85 | 7.34 | 3.29 |
| Found: | 81.64 | 7.58 | 3.24 |

Example 20

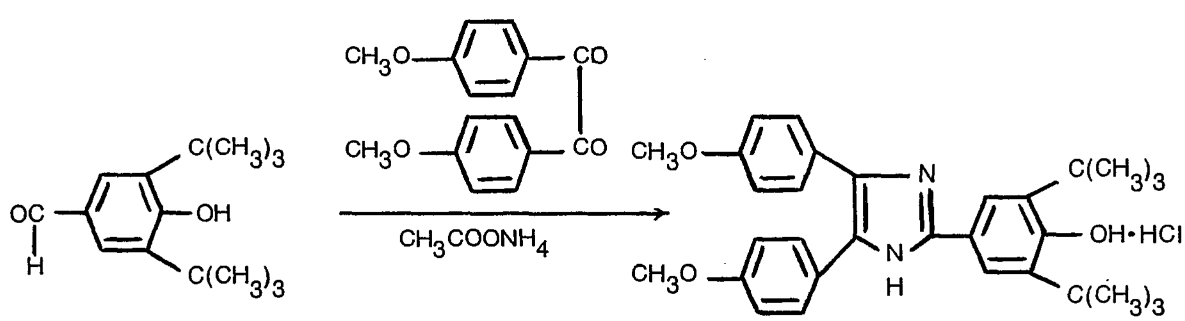


In 20 ml of acetic acid was dissolved 1.4 g of p-anisil and after adding thereto 1.2 g of 3,5-di-tert-butyl-4-hydroxybenzaldehyde and 5 g of ammonium acetate, the mixture was refluxed for one hour. To the

reaction mixture was added 10 ml of water and the crystals thus precipitated were recovered by filtration and dissolved in 30 ml of chloroform. After washing the chloroform solution with dilute aqueous ammonia, the solvent was distilled off. The crystals thus obtained were washed with water and then methanol, treated with hydrogen chloride-containing methanol, and recrystallized from a mixture of methanol and ether to provide 1.4 g of 2-(3,5-di-tert-butyl-4-hydroxyphenyl)-4,5-bis(p-methoxyphenyl)imidazole hydrochloride.

Melting point: 292—295°C
Elemental analysis for $C_{31}H_{37}N_2O_3Cl$:

| | C(%) | H(%) | N(%) |
|---|---|---|---|
| Calculated: | 71.45 | 7.16 | 5.35 |
| Found: | 71.08 | 7.23 | 5.38 |

Example 21

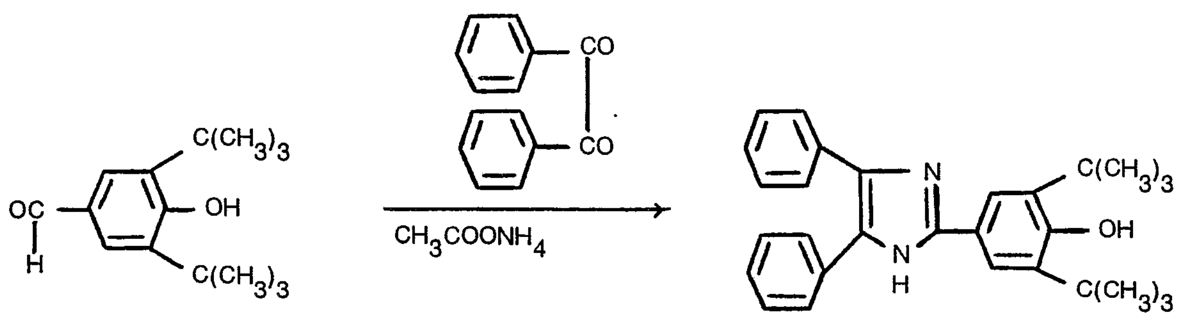


By following the same procedure as in Example 20 using 1.05 g of benzil, 1.8 g of 2-(3,5-di-tert-butyl-4-hydroxyphenyl)-4,5-diphenylimidazole hydrochloride was obtained.

Melting point: above 300°C
Elemental analysis for $C_{29}H_{33}N_2OCl$:

| | C(%) | H(%) | N(%) . |
|---|---|---|---|
| Calculated: | 75.55 | 7.21 | 6.08 |
| Found: | 75.38 | 7.32 | 6.07 |

Example 22

In 60 ml of ethanol was suspended 10 g of ammonium amidodithiocarbonate and 15 g of α-bromo-3,5-di-tert-butyl-4-hydroxyacetophenone was added portionwise to the suspension under stirring of 0—5°C. Then, after stirring the mixture overnight at room temperature, 200 ml of water was added to the reaction mixture and the product was extracted with benzene. The extract was concentrated and the residue was dissolved in 50 ml of acetic acid and refluxed for 4 hours. To the reaction mixture were added 150 ml of water and 50 ml of ethyl acetate and then the mixture was stirred. The crystals thus precipitated were recovered by filtration and recrystallized from dioxane to provide 9.5 g of 4-(3,5-di-tert-butyl-4-hydroxyphenyl)-2-mercaptothiazole as the 1/2 dioxane addition product thereof.

Melting point: 285—288°C
Elemental analysis for $C_{19}H_{27}O\ NS_2$:

| | C(%) | H(%) | N(%) |
|---|---|---|---|
| Calculated: | 62.43 | 7.44 | 3.83 |
| Found: | 62.31 | 7.47 | 3.96 |

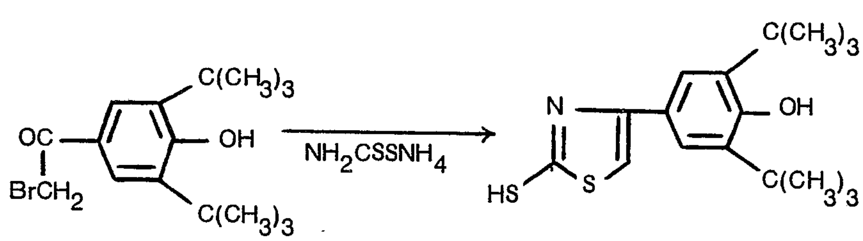

Example 23

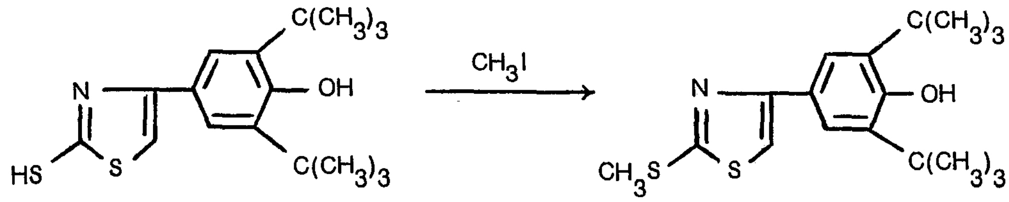


In 30 ml of methanol was dissolved 0.18 g of metallic sodium and after adding thereto 2.1 g of 4-(3,5-di-tert-butyl-4-hydroxyphenyl)-2-mercaptothiazole, 1.15 g of methyliodide was added dropwise to the solution at 0—5°C. After allowing the mixture to stand for 30 minutes at room temperature, 60 ml of iced water was added to the reaction mixture. The crystals thus precipitated were recovered by filtration and recrystallized from n-hexane to provide 1.6 g of 4-(3,5-di-tert-butyl-4-hydroxyphenyl)-2-methylthiothiazole.

Melting point: 92—93°C
Elemental analysis for $C_{18}H_{25}NOS_2$:

| | C(%) | H(%) | N(%) | S(%) |
|---|---|---|---|---|
| Calculated: | 64.44 | 7.51 | 4.17 | 19.11 |
| Found: | 64.36 | 7.69 | 4.23 | 19.28 |

Example 24

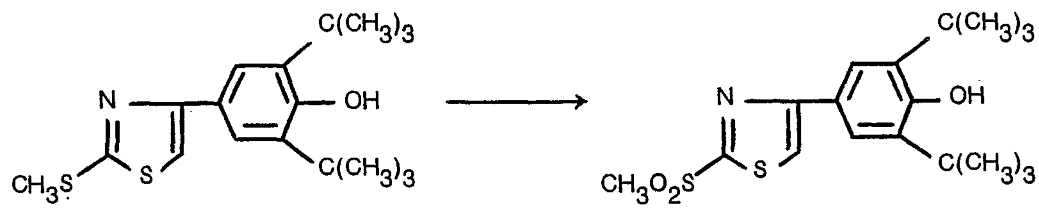

In 30 ml of chloroform was dissolved 0.8 g of 4-(3,5-di-tert-butyl-4-hydroxyphenyl)-2-methylthiothiazole and then 1.5 g of m-chloroperbenzoic acid was added portionwise to the solution with stirring. After one hour, the reaction mixture was washed with a 5% sodium hydrogen carbonate solution, and the chloroform layer was concentrated. The crystals obtained were recrystallized from methanol to provide 0.55 g of 3,5-di-tert-butyl-4-hydroxyphenyl-2-methylsulfonylthiazole.

Melting point: 183—184°C
Elemental analysis for $C_{18}H_{25}NO_3S_2$:

| | C(%) | H(%) | N(%) | S(%) |
|---|---|---|---|---|
| Calculated: | 58.83 | 6.86 | 3.81 | 17.45 |
| Found: | 58.72 | 7.00 | 3.69 | 17.66 |

Example 25

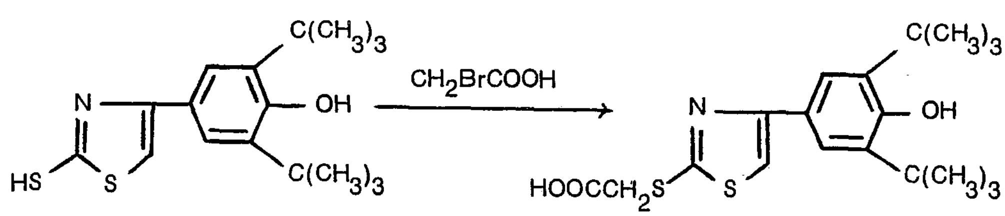


To 20 ml of benzene were added 0.64 g of 4-(3,5-di-tert-butyl-4-hydroxyphenyl)-2-mercaptothiazole and 0.32 g of bromoacetic acid and then 0.5 g of triethylamine was added dropwise to the mixture with stirring. After one hour triethylamine hydrobromide thus precipitated was filtered off and the benzene solution was extracted with 10 ml of a 2% sodium hydroxide solution. The aqueous extract was acidified by the addition of hydrochloric acid and extracted with ether. The ether solution was dried over anhydrous sodium sulfate

and concentrated. The crystals thus obtained were recrystallized from benzene to provide 0.52 g of [4-(3,5-di-tert-butyl-4-hydroxyphenyl)-2-thiazolylthio]acetic acid.

Melting point: 158—159°C
Elemental analysis for $C_{19}H_{25}NO_3S_2$:

| | C(%) | H(%) | N(%) | S(%) |
|---|---|---|---|---|
| Calculated: | 60.13 | 6.64 | 3.69 | 16.89 |
| Found: | 60.06 | 6.62 | 3.77 | 16.69 |

Example 26

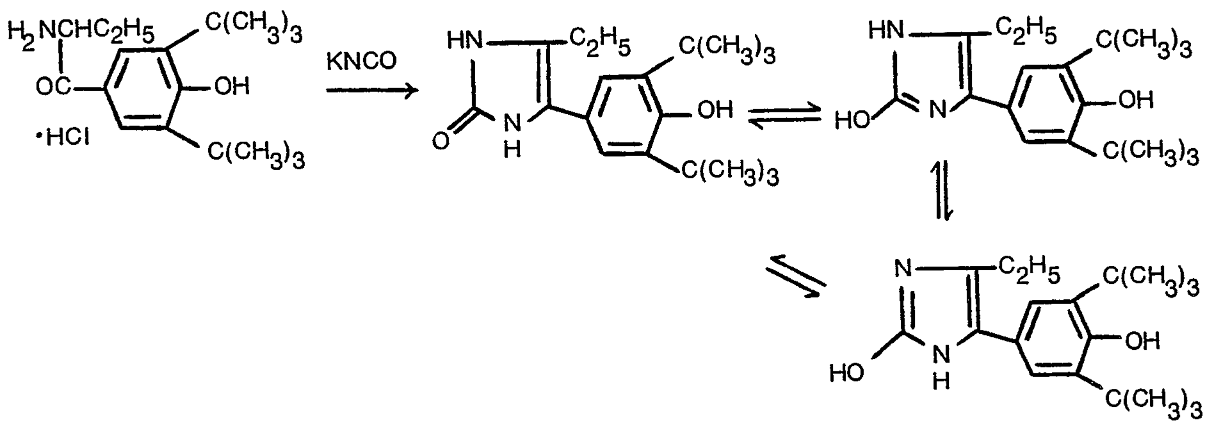


By following the same procedure as in Example 6 using 3.3 g of 2-amino-1-(3,5-di-tert-butyl-4-hydroxyphenyl)-1-butanone hydrochloride, 1.62 g of potassium isocyanate, and 1.6 ml of concentrated hydrochloric acid and recrystallizing the reaction product from aqueous isopropanol, 1.6 g of 4-(3,5-di-tert-butyl-4-hydroxyphenyl)-5-ethyl-2-oxo-4-imidazoline was obtained.

Melting point: 267—270°C
Elemental analysis for $C_{19}H_{28}N_2O_2$:

| | C(%) | H(%) | N(%) |
|---|---|---|---|
| Calculated: | 72.12 | 8.92 | 8.85 |
| Found: | 71.83 | 9.17 | 8.62 |

Example 27

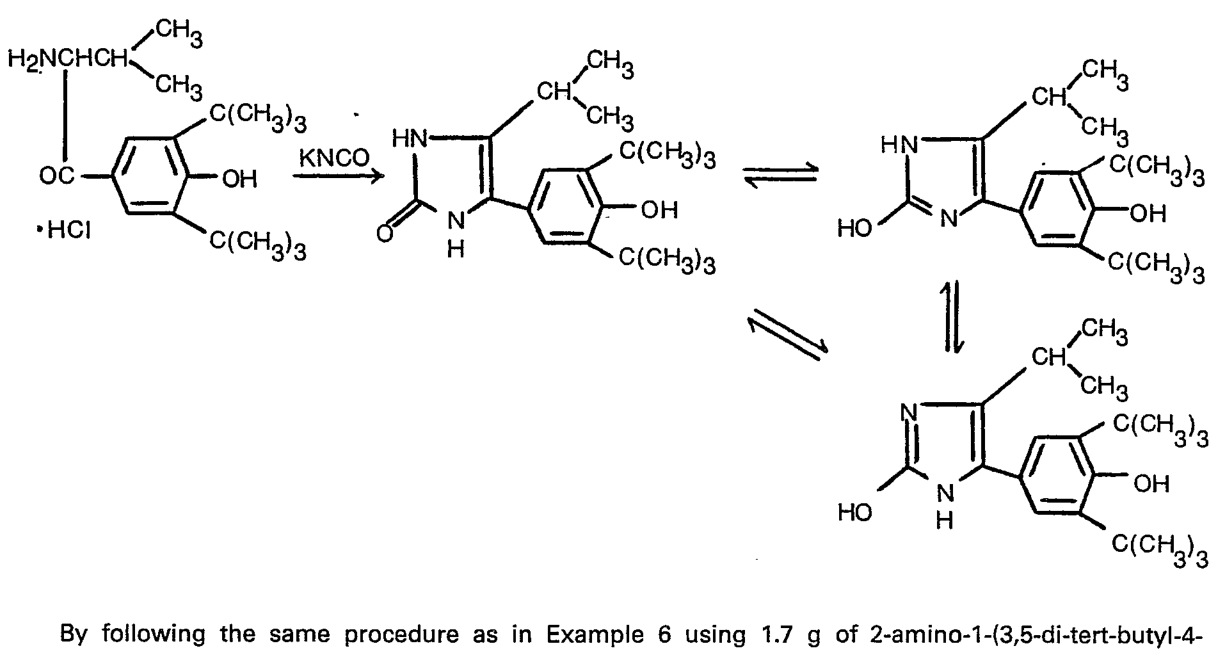


By following the same procedure as in Example 6 using 1.7 g of 2-amino-1-(3,5-di-tert-butyl-4-hydroxyphenyl)-3-methyl-1-butanone hydrochloride, 0.8 g of potassium isocyanate, and 1 ml of concentrated hydrochloric acid and recrystallizing the reaction product from aqueous isopropanol, 0.35 g of 4-(3,5-di-tert-butyl-4-hydroxyphenyl)-5-isopropyl-2-oxo-4-imidazoline was obtained.

Melting point: above 290°C (decompd)
Elemental analysis for $C_{20}H_{30}N_2O_2$:

| | C(%) | H(%) | N(%) |
|---|---|---|---|
| Calculated: | 72.69 | 9.15 | 8.48 |
| Found: | 72.37 | 9.26 | 8.47 |

Example 28

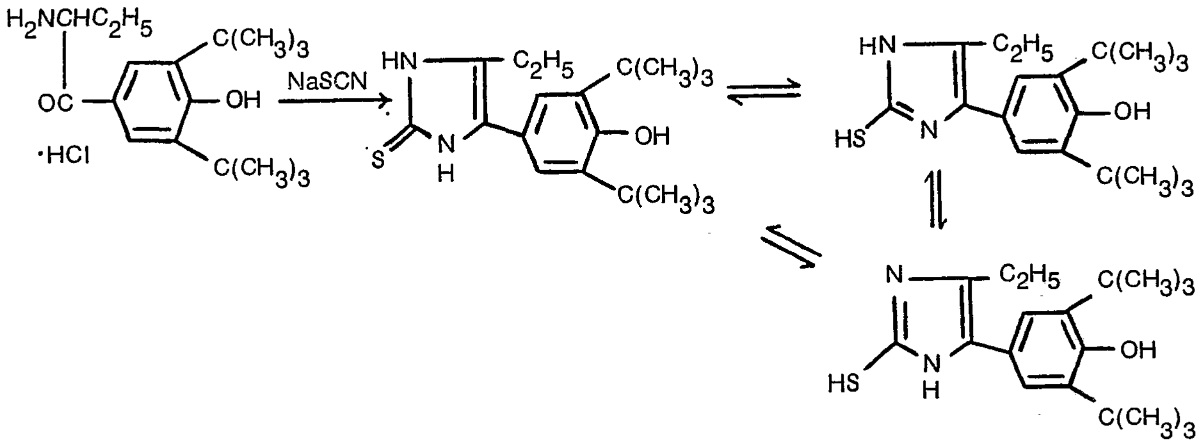


By following the same procedure as in Example 8 using 13.1 g of 2-amino-1-(3,5-di-tert-butyl-4-hydroxyphenyl)-1-butanone hydrochloride, 6.5 g of sodium thiocyanate, 3.4 ml of concentrated HCl and 100 ml of acetic acid and recrystallizing the reaction product from isopropanol, 6 g of 4-(3,5-di-tert-butyl-4-hydroxyphenyl)-5-ethyl-2-thioxo-4-imidazoline was obtained.

Melting point: 279—281°C
Elemental analysis for $C_{19}H_{28}N_2OS+(CH_3)_2CHOH$:

| | C(%) | H(%) | N(%) |
|---|---|---|---|
| Calculated: | 67.31 | 9.24 | 7.14 |
| Found: | 67.17 | 9.36 | 7.29 |

Example 29

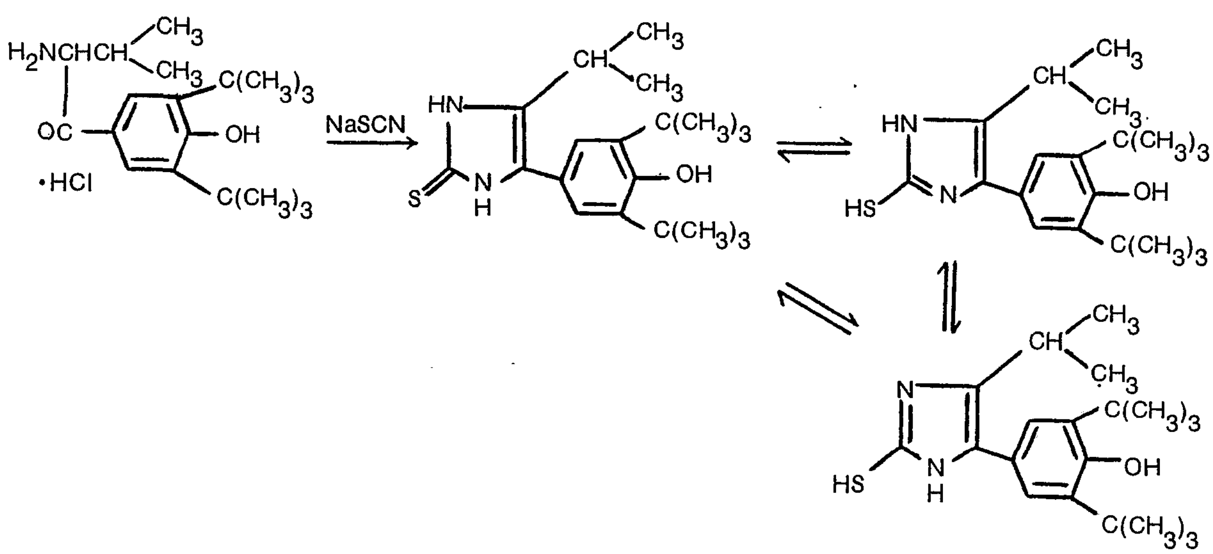


By following the same procedure as in Example 8 using 3.4 g of 2-amino-1-(3,5-di-tert-butyl-4-hydroxyphenyl)-3-methyl-1-butanone hydrochloride, 1.6 g of sodium thiocyanate, 1 ml of concentrated hydrochloric acid, and 31 ml of acetic acid and recrystallizing the reaction product from water-containing isopropanol, 0.8 g of 4-(3,5-di-tert-butyl-4-hydroxyphenyl)-5-isopropyl-2-thioxo-4-imidazoline was obtained.

Melting point: above 300°C (decomposed)
Elemental analysis for $C_{20}H_{30}N_2OS$:

| | C(%) | H(%) | N(%) |
|---|---|---|---|
| Calculated: | 69.32 | 8.73 | 8.08 |
| Found: | 69.18 | 8.95 | 7.74 |

Example 30

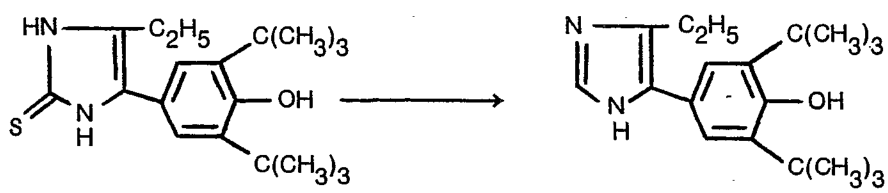

By following the same procedure as in Example 17 using 2 g of 4-(3,5-di-tert-butyl-4-hydroxyphenyl)-5-ethyl-2-thioxo-4-imidazoline, 50 ml of absolute ethanol, and 1 g of a Raney nickel catalyst and recrystallizing the reaction product from toluene, 0.9 g of 4-(3,5-di-tert-butyl-4-hydroxyphenyl)-5-ethyl-imidazole was obtained.

Melting point: 200—201°C
Elemental analysis for $C_{19}H_{28}N_2O+1/2C_7H_8$:

| | C(%) | H(%) | N(%) |
|---|---|---|---|
| Calculated: | 77.99 | 9.31 | 8.08 |
| Found: | 77.72 | 9.53 | 8.13 |

Example 31

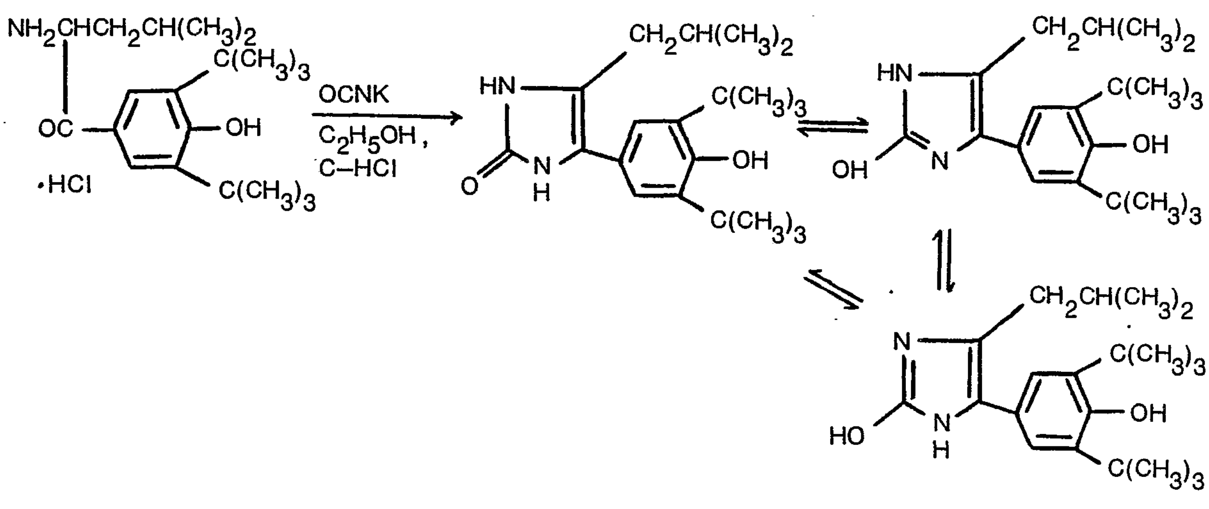


By following the same procedure as in Example 6, 4-(3,5-di-tert-butyl-4-hydroxyphenyl)-5-isobutyl-2-oxo-4-imidazoline was produced.

Melting point: 286—289°C (benzene-hexane)
Elemental analysis for $C_{21}H_{32}N_2O_2$:

| | C(%) | H(%) | N(%) |
|---|---|---|---|
| Calculated: | 73.22 | 9.36 | 8.13 |
| Found: | 73.43 | 9.57 | 8.18 |

Examples 32—38

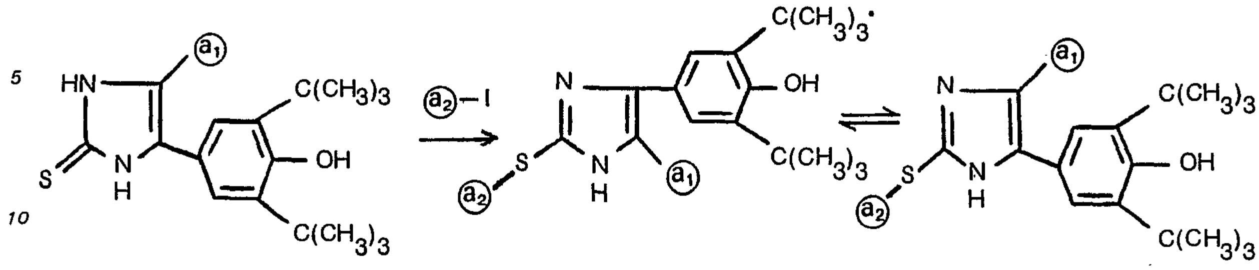

By following the same procedure as in Example 10, the following compounds were produced.

Example 32

Compound: 4-(3,5-di-tert-butyl-4-hydroxyphenyl)-2-ethylthio-5-methylimidazole.

( $a_1$ = $CH_3$, $a_2$ = $CH_2CH_3$)

Melting point: 245—250°C (toluene)
Elemental analysis for $C_{20}H_{30}N_2OS$:

| | C(%) | H(%) | N(%) | S(%) |
|---|---|---|---|---|
| Calculated: | 69.32 | 8.73 | 8.08 | 9.25 |
| Found: | 69.38 | 8.70 | 8.00 | 9.47 |

Example 33

Compound: 4-(3,5-di-tert-butyl-4-hydroxyphenyl)-2-isopropylthio-5-methylimidazole.

( $a_1$ = $CH_3$, $a_2$ = 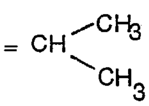 )

Melting point: 250—258°C (toluene)
Elemental analysis for $C_{21}H_{32}N_2OS$:

| | C(%) | H(%) | N(%) | S(%) |
|---|---|---|---|---|
| Calculated: | 69.96 | 8.95 | 7.77 | 8.89 |
| Found: | 69.74 | 9.02 | 7.61 | 8.63 |

Example 34

Compound: 4-(3,5-di-tert-butyl-4-hydroxyphenyl)-5-ethyl-2-methylthioimidazole.

( $a_1$ = $CH_2CH_3$, $a_2$ = $CH_3$)

Melting point: 210—211°C (toluene)
Elemental analysis for $C_{20}H_{30}N_2OS$:

| | C(%) | H(%) | N(%) |
|---|---|---|---|
| Calculated: | 69.32 | 8.73 | 8.08 |
| Found: | 69.05 | 8.68 | 7.48 |

Example 35

Compound: 4-(3,5-di-tert-butyl-4-hydroxyphenyl)-5-ethyl-2-ethylthioimidazole.

( $a_1$ = $CH_2CH_3$, $a_2$ = $CH_2CH_3$)

Melting point: 226—228°C (toluene)

Elemental analysis for $C_{21}H_{32}N_2OS$:

| | C(%) | H(%) | N(%) |
|---|---|---|---|
| Calculated: | 69.96 | 8.95 | 7.74 |
| Found: | 70.18 | 8.97 | 7.63 |

Example 36

Compound: 4-(3,5-di-tert-butyl-4-hydroxyphenyl)-5-ethyl-2-isopropylthioimidazole.

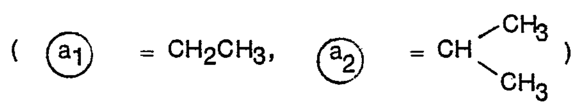

Melting point: 247—248°C (toluene)
Elemental analysis for $C_{22}H_{34}N_2OS$:

| | C(%) | H(%) | N(%) |
|---|---|---|---|
| Calculated: | 70.54 | 9.15 | 7.48 |
| Found: | 70.32 | 9.27 | 7.32 |

Example 37

Compound: 4-(3,5-di-tert-butyl-4-hydroxyphenyl)-5-isopropyl-2-methylthioimidazole.

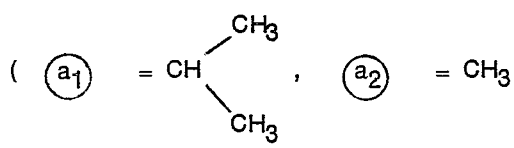

Melting point: 222—223°C (ethyl acetate-hexane)
Elemental analysis for $C_{21}H_{32}N_2OS$:

| | C(%) | H(%) | N(%) |
|---|---|---|---|
| Calculated: | 69.96 | 8.95 | 7.77 |
| Found: | 69.78 | 9.01 | 7.65 |

Example 38

Compound: 4-(3,5-di-tert-butyl-4-hydroxyphenyl)-5-isobutyl-2-methylthioimidazole.

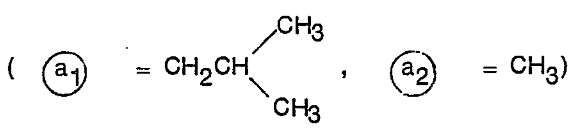

Melting point: 210—212°C (benzene-hexane)
Elemental analysis for $C_{22}H_{34}N_2OS$:

| | C(%) | H(%) | N(%) |
|---|---|---|---|
| Calculated: | 70.54 | 9.15 | 7.48 |
| Found: | 70.45 | 9.24 | 7.07 |

Example 39

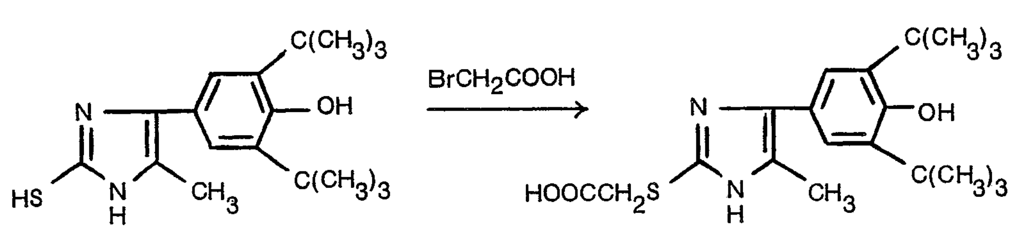


In 30 ml of toluene were dissolved 1.6 g of 4-(3,5-di-tert-butyl-4-hydroxyphenyl)-5-methyl-2-thioxo-4-imidazoline and 0.7 g of bromoacetic acid and after adding thereto 1 g of triethylamine at room

temperature, the mixture was refluxed for 4 hours. After cooling, the reaction mixture was filtered and the filtrate was extracted with an aqueous sodium carbonate solution and the alkali solution was acidified with dilute hydrochloric acid. The precipitates thus formed were recovered by filtration and recrystallized from aqueous isopropanol to provide 0.5 g of [4-(3,5-di-tert-butyl-4-hydroxyphenyl)-5-methyl-2-imidazoylthio]acetic acid.

Melting point: 252—254°C
Elemental analysis for $C_{20}H_{28}N_2O_3S$:

| | C(%) | H(%) | N(%) |
|---|---|---|---|
| Calculated: | 63.80 | 7.50 | 7.44 |
| Found: | 63.53 | 7.74 | 7.26 |

Example 40

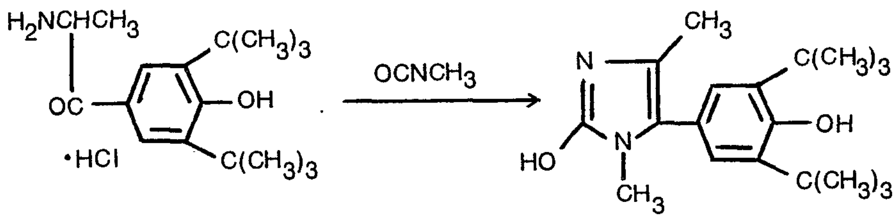


By following the same procedure as in Example 16, 5-(3,5-di-tert-butyl-4-hydroxyphenyl)-1,4-dimethyl-2-oxo-4-imidazoline was produced.

Melting point: 285—286°C
Elemental analysis for $C_{19}H_{28}N_2O_2$:

| | C(%) | H(%) | N(%) |
|---|---|---|---|
| Calculated: | 72.12 | 8.92 | 8.85 |
| Found: | 72.18 | 9.18 | 8.57 |

Examples 41—42

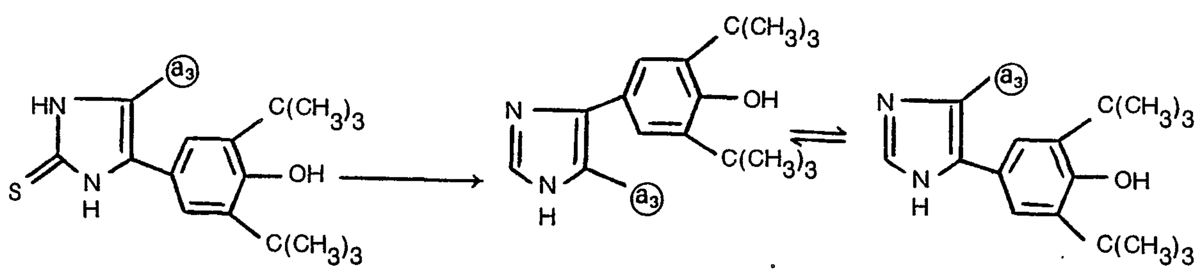

By following the same procedure as in Example 17, the following compounds were produced

Example 41
Compound: 4-(3,5-di-tert-butyl-4-hydroxyphenyl)-5-isopropylimidazole.1/2$CH_3OH$

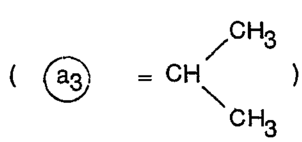

Melting point: 208—210°C (methanol)
Elemental analysis for $C_{20}H_{30}N_2O.1/2CH_3OH$:

| | C(%) | H(%) | N(%) |
|---|---|---|---|
| Calculated: | 74.50 | 9.76 | 8.48 |
| Found: | 74.23 | 10.08 | 8.43 |

Example 42

Compound: 4-(3,5-di-tert-butyl-4-hydroxyphenyl)-5-isobutylimidazole.

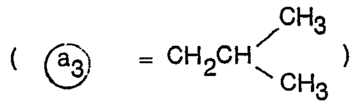

Melting point: 202—203°C (benzene)

Elemental analysis for $C_{21}H_{32}N_2O$:

| | C(%) | H(%) | N(%) |
|---|---|---|---|
| Calculated: | 76.78 | 9.82 | 8.53 |
| Found: | 76.64 | 9.95 | 8.45 |

Example 43

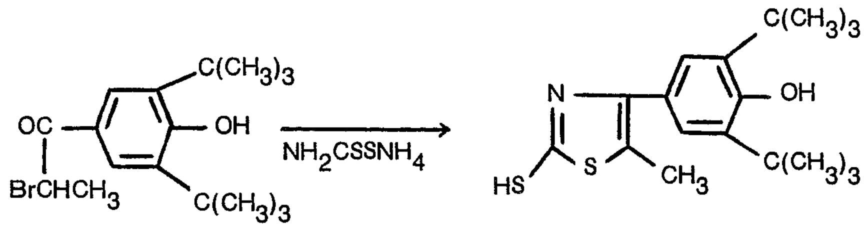


By following the same procedure as in Example 22, 4-(3,5-di-tert-butyl-4-hydroxyphenyl)-2-mercapto-5-methylthiazole was produced.

Melting point: 267—269°C (tetrahydrofuran-hexane)

Elemental analysis for $C_{18}H_{25}NOS_2$:

| | C(%) | H(%) | N(%) |
|---|---|---|---|
| Calculated: | 64.44 | 7.51 | 4.17 |
| Found: | 64.29 | 7.50 | 4.16 |

Examples 44—49

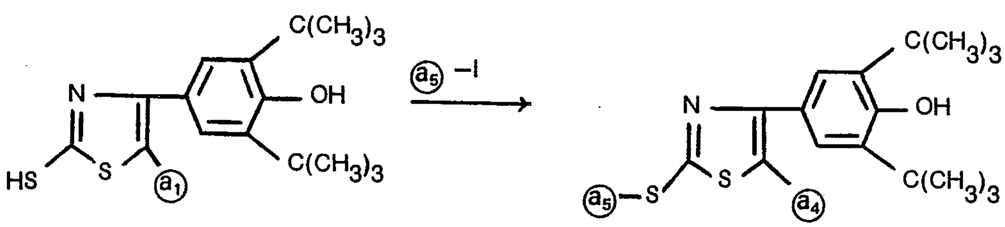

By following the same procedure as in Example 23, the following compounds were produced.

Example 44

Compound: 4-(3,5-di-tert-butyl-4-hydroxyphenyl)-2-ethylthiothiazole.

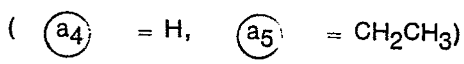

Melting point: 130—131°C (methanol)

Elemental analysis for $C_{19}H_{27}NOS_2$:

| | C(%) | H(%) | N(%) | S(%) |
|---|---|---|---|---|
| Calculated: | 65.29 | 7.79 | 4.01 | 18.34 |
| Found: | 64.97 | 7.99 | 4.01 | 18.38 |

Example 45

Compound: 4-(3,5-di-tert-butyl-4-hydroxyphenyl)-2-isopropylthiothiazole.

( $a_4$ = H, $a_5$ = 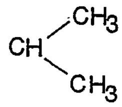 )

Melting point: 173—174°C (hexane)

Elemental analysis for $C_{20}H_{29}NOS_2$:

| | C(%) | H(%) | N(%) | S(%) |
|---|---|---|---|---|
| Calculated: | 66.07 | 8.04 | 3.85 | 17.64 |
| Found: | 66.20 | 7.84 | 3.67 | 17.80 |

Example 46

Compound: 4-(3,5-di-tert-butyl-4-hydroxyphenyl)-5-methyl-2-methylthiothiazole.

( $a_4$ = $CH_3$, $a_5$ = $CH_3$)

Melting point: 118—119°C (hexane)

Elemental analysis for $C_{19}H_{27}NOS_2$:

| | C(%) | H(%) | N(%) | S(%) |
|---|---|---|---|---|
| Calculated: | 65.29 | 7.79 | 4.01 | 18.34 |
| Found: | 65.41 | 7.96 | 4.01 | 18.60 |

Example 47

Compound: 4-(3,5-di-tert-butyl-4-hydroxyphenyl)-5-ethyl-2-methylthiothiazole.

( $a_4$ = $CH_2CH_3$, $a_5$ = $CH_3$)

Melting point: 115—116°C (hexane)

Elemental analysis for $C_{20}H_{29}NOS_2$:

| | C(%) | H(%) | N(%) | S(%) |
|---|---|---|---|---|
| Calculated: | 66.07 | 8.04 | 3.85 | 17.64 |
| Found: | 66.17 | 8.18 | 4.11 | 17.60 |

Example 48

Compound: 4-(3,5-di-tert-butyl-4-hydroxyphenyl)-5-isopropyl-2-methylthiothiazole.

( $a_4$ = 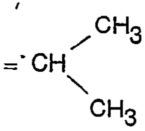 , $a_5$ = $CH_3$)

Melting point: 135—137°C (methanol)

Elemental analysis for $C_{21}H_{31}NOS_2$:

| | C(%) | H(%) | N(%) | S(%) |
|---|---|---|---|---|
| Calculated: | 66.80 | 8.27 | 3.71 | 16.98 |
| Found: | 66.70 | 8.37 | 3.77 | 17.20 |

Example 49

Compound: 4-(3,5-di-tert-butyl-4-hydroxyphenyl)-5-isobutyl-2-methylthiothiazole.

( $a_4$ = 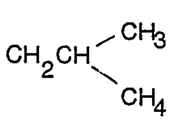 , $a_5$ = $CH_3$)

Melting point: 96—97°C (methanol)
Elemental analysis for $C_{22}H_{33}NOS_2$:

| | C(%) | H(%) | N(%) | S(%) |
|---|---|---|---|---|
| Calculated: | 67.42 | 8.49 | 3.58 | 16.37 |
| Found: | 67.12 | 8.71 | 3.58 | 16.59 |

Example 50

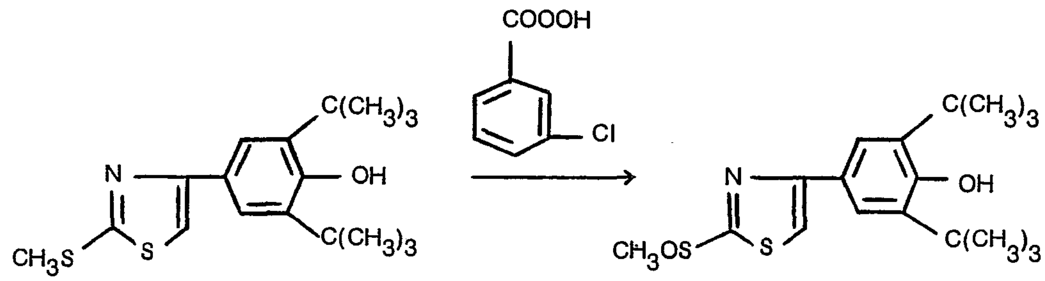

In 5 ml of chloroform was dissolved 0.8 g of 4-(3,5-di-tert-butyl-4-hydroxyphenyl)-2-methylthiothiazole and then 0.4 g of m-chloroperbenzoic acid (80%) was added portionwise to the solution with stirring. After 30 minutes, the reaction mixture was washed with an aqueous 5% sodium hydrogencarbonate solution and the chloroform layer was concentrated. The crystals thus obtained were recrystallized from hexane to provide 0.45 g of 4-(3,5-di-tert-butyl-4-hydroxyphenyl)-2-methylsulfinylthiazole.

Melting point: 124—125°C
Elemental analysis for $C_{18}H_{25}NO_2S_2$:

| | C(%) | H(%) | N(%) | S(%) |
|---|---|---|---|---|
| Calculated: | 61.50 | 7.17 | 3.98 | 18.24 |
| Found: | 61.41 | 7.32 | 4.05 | 18.56 |

Example 51

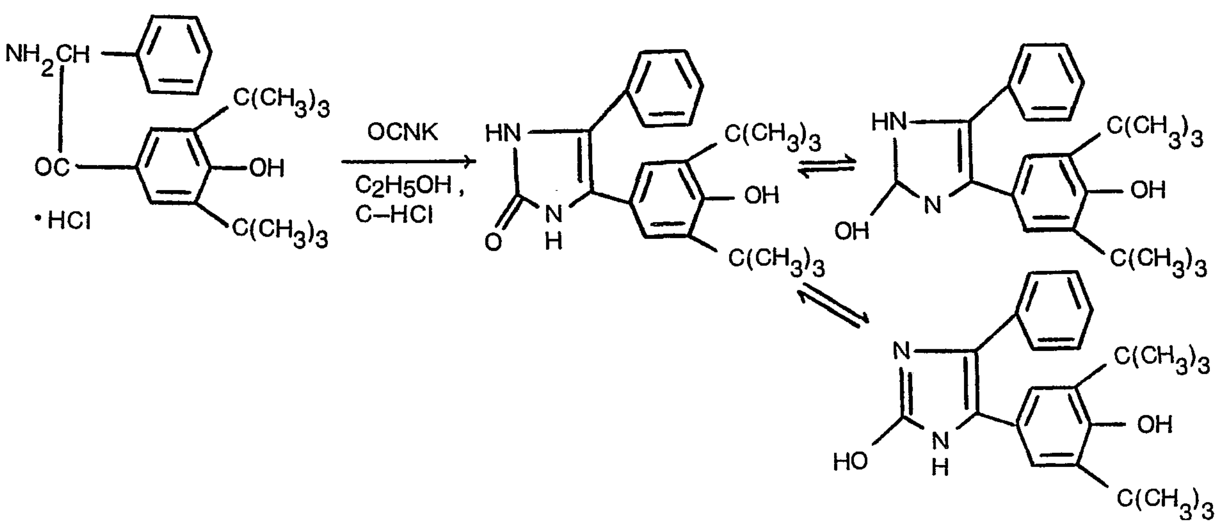


By following the same procedure as in Example 6, 4-(3,5-di-tert-butyl-4-hydroxyphenyl)-5-phenyl-2-oxo-imidazoline was produced.

Melting point: >300°C (DMF-water)
Elemental analysis for $C_{23}H_{28}N_2O_2$:

| | C(%) | H(%) | N(%) |
|---|---|---|---|
| Calculated: | 75.79 | 7.74 | 7.69 |
| Found: | 75.70 | 7.97 | 7.85 |

Example 52

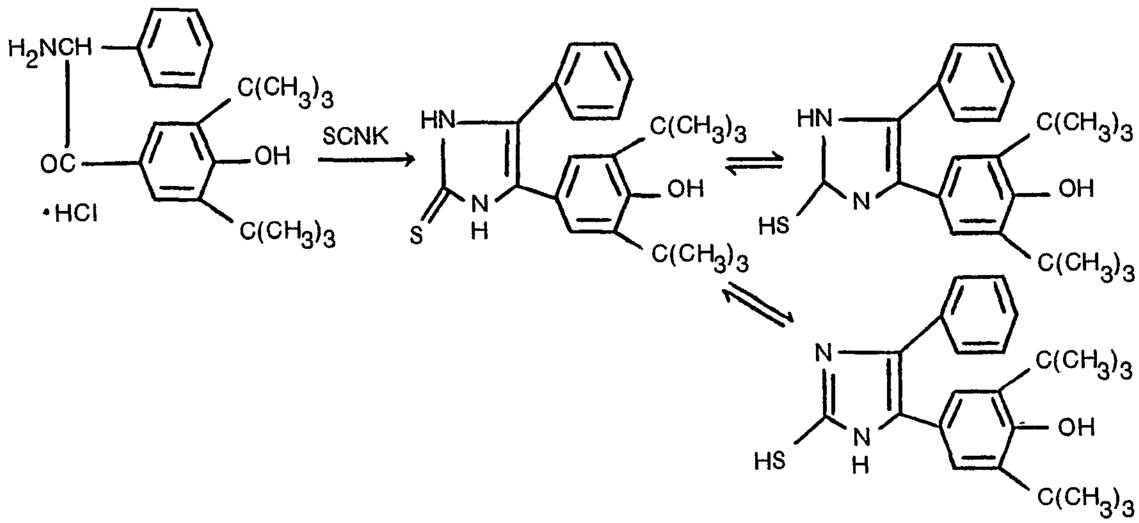


By following the same procedure as in Example 8, 4-(3,5-di-tert-butyl-4-hydroxyphenyl)-5-phenyl-2-thioxo-4-imidazoline was produced.

Melting point: 258—262°C (toluene)
Elemental analysis for $C_{23}H_{28}N_2OS$:

| | C(%) | H(%) | N(%) | S(%) |
|---|---|---|---|---|
| Calculated: | 72.59 | 7.42 | 7.36 | 8.42 |
| Found: | 72.70 | 7.52 | 7.49 | 8.28 |

Example 53

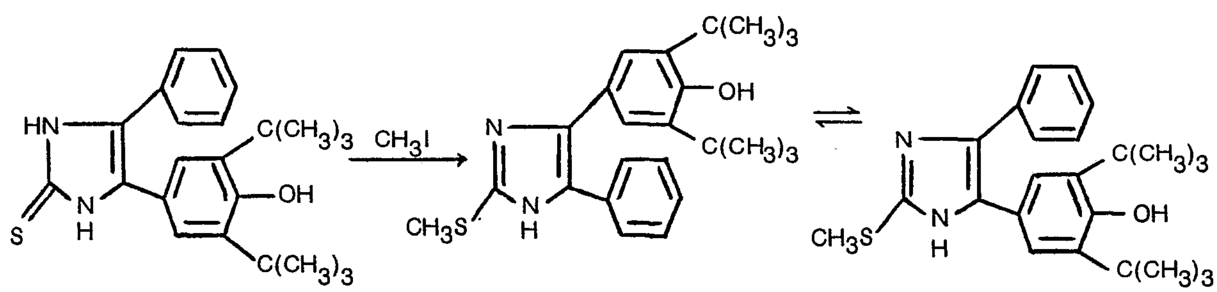


By following the same procedure as in Example 10, 4-(3,5-di-tert-butyl-4-hydroxyphenyl)-2-methylthio-5-phenylimidazole was produced.

Melting point: 129—131°C (toluene-hexane)
Elemental analysis for $C_{24}H_{30}N_2OS.1/4C_7H_8$:

| | C(%) | H(%) | N(%) | S(%) |
|---|---|---|---|---|
| Calculated: | 74.10 | 7.67 | 6.71 | 7.67 |
| Found: | 73.64 | 7.86 | 6.42 | 7.46 |

Example 54

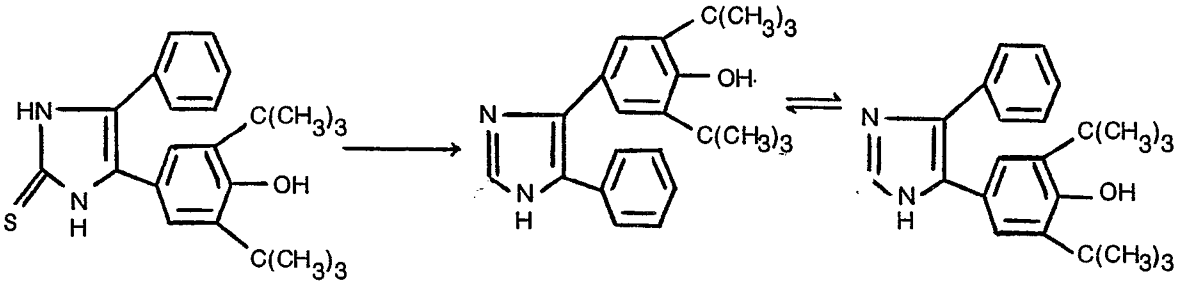

By following the same procedure as in Example 17, 4-(3,5-di-tert-butyl-4-hydroxyphenyl)-5-phenylimidazole was produced.

Melting point: 170—171°C (toluene)
Elemental analysis for $C_{23}H_{28}N_2O$:

| | C(%) | H(%) | N(%) |
|---|---|---|---|
| Calculated: | 79.27 | 8.10 | 8.04 |
| Found: | 79.16 | 8.28 | 8.06 |

Example 55

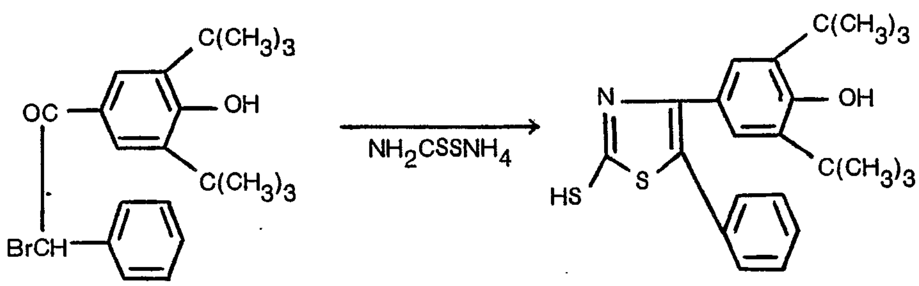


By following the same procedure as in Example 22, 4-(3,5-di-tert-butyl-4-hydroxyphenyl)-2-mercapto-5-phenylthiazole was produced.

Melting point: 168—171°C (tetrahydrofuran-hexane)
Elemental analysis for $C_{23}H_{27}NOS_2$:

| | C(%) | H(%) | N(%) | S(%) |
|---|---|---|---|---|
| Calculated: | 69.48 | 6.84 | 3.52 | 16.13 |
| Found: | 69.80 | 7.10 | 3.43 | 16.22 |

Example 56

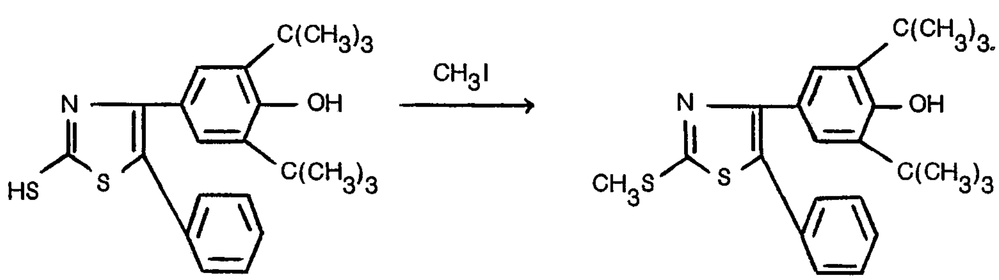


By following the same procedure as in Example 23, 4-(3,5-di-tert-butyl-4-hydroxyphenyl)-2-methylthio-5-phenylthiazole was produced.

Melting point: 135—137°C (hexane)

Elemental analysis for $C_{24}H_{29}NOS_2$:

| | C(%) | H(%) | N(%) | S(%) |
|---|---|---|---|---|
| Calculated: | 70.03 | 7.10 | 3.40 | 15.58 |
| Found: | 70.30 | 7.37 | 3.44 | 15.61 |

Example 57

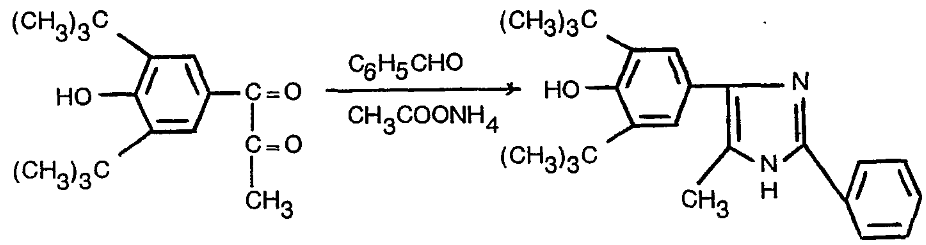


In 10 ml of acetic acid was dissolved 0.6 g of 1-(3,5-di-tert-butyl-4-hydroxyphenyl)-1,2-propanedione and then after adding thereto 0.3 g of benzaldehyde and 2.2 g of ammonium acetate, the mixture was refluxed for 3 hours. The reaction mixture was concentrated and the residue formed was mixed with 20 ml of water, neutralized by the addition of concentrated aqueous ammonia, and extracted with chloroform. The extract was dried and concentrated under reduced pressure. The crystals thus formed were purified by silica gel column chromatography using chloroform as the eluent and recrystallized from aqueous ethanol to provide 0.3 g of 4-(3,5-di-tert-butyl-4-hydroxyphenyl)-5-methyl-2-phenylimidazole.

Melting point: 298—300°C

Elemental analysis for $C_{24}H_{30}N_2O$:

| | C(%) | H(%) | N(%) |
|---|---|---|---|
| Calculated: | 79.52 | 8.34 | 7.73 |
| Found: | 79.67 | 8.45 | 7.77 |

The clinical doses of the compounds of this invention are usually 10—1,000 mg per day for an adult and the medicament is administered 2 or 3 times per day. The doses are controlled according to the condition and age of the patient.

The compounds of this invention can be administered in various dosage forms such as formulations for oral administration, injections, suppositories for rectal administration, and medicines for topical application.

The compounds of this invention may be used in pharmaceutical compositions prepared by blending with conventional pharmaceutical carriers or excipients in conventional manner. The tablets, capsules, granules, powders, etc., of the compounds of this invention for oral administration may contain conventional pharmaceutical excipients, such as calcium carbonate, calcium phosphate, starch, sucrose, lactose, talc, magnesium stearate, gelatin, polyvinyl pyrrolidone, gum arabic, sorbitol, microcrystalline cellulose, polyethylene glycol, silica, and sodium laurylsulfate. Moreover, the tablets may be coated as well known in the art. Furthermore, the liquid formulations for oral administration may be aqueous or oily suspensions, syrups, or elixirs, prepared in conventional manner. Suppositories for rectal use may contain a formulation carrier known in the art, such as polyethylene glycol, lanolin, cacao butter or WITEPSOL (TM) (made by Dynamite Nobel Co.)

**Claim:**

1. A process of producing a 3,5-di-tert-butyl-4-hydroxyphenyl-substituted heterocyclic compound represented by the following formula Ia—1 or a salt thereof:

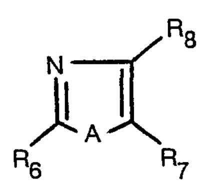

Ia—1

wherein one of $R_6$, $R_7$ and $R_8$ is the 3,5-di-tert-butyl-4-hydroxyphenyl group and the others are the same or different and selected from hydrogen, $C_1$ to $C_7$ alkyl groups, aryl-substituted $C_1$ to $C_7$ alkyl groups, aryl groups, $C_1$ to $C_7$ alkoxy-substituted aryl groups, and the groups —O—Z,

$$-\underset{(O)_n}{S}-Z,$$

and —NH—Z (wherein Z represents hydrogen, a $C_1$ to $C_7$ alkyl group, a carboxy $C_1$ to $C_7$ alkyl group, a ($C_1$ to $C_7$ alboxy) carbonyl $C_1$ to $C_7$ alkyl group, an aryl-substituted $C_1$ to $C_7$ alkyl group, or an aryl group and n is 0, 1, or 2); A is oxygen, sulfur, or >NR where R is hydrogen or a $C_1$ to $C_7$ alkyl or 3,5-di-tert-butyl-4-hydroxyphenacyl group which comprises reacting a compound represented by formula IIa

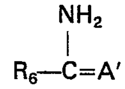
IIa

wherein A′ is oxygen, sulfur, an imino group or a $C_1$ to $C_7$ alkylimino group and $R_6$ is as defined above with a halogenocarbonyl compound represented by formula IIIa

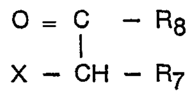
IIIa

wherein X is halogen and $R_7$ and $R_8$ are as defined above, and then, if desired, oxidising, alkylating or disulfurising the resulting product.

2. A process of producing a 3,5-di-tert-butyl-4-hydroxyphenyl-substituted heterocyclic compound represented by the following formula Ia—2 or a salt thereof:

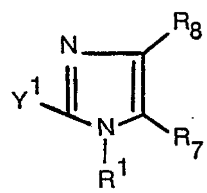
Ia—2

wherein one of $R_7$ and $R_8$ is the 3,5-di-tert-butyl-4-hydroxyphenyl group and the other is the same or different and selected from hydrogen, $C_1$ to $C_7$ alkyl groups, aryl-substituted $C_1$ to $C_7$ alkyl groups, aryl groups, $C_1$ to $C_7$ alkoxy-substituted aryl groups, and the groups —O—Z,

$$-\underset{(O)_n}{S}-Z,$$

and —NH—Z (wherein Z represents hydrogen, a $C_1$ to $C_7$ alkyl group, a carboxy $C_1$ to $C_7$ alkyl group, a ($C_1$ to $C_7$ alkoxy) carbonyl $C_1$ to $C_7$ alkyl group, an aryl-substituted $C_1$ to $C_7$ alkyl group, or an aryl group and n is 0, 1, or 2); $R^1$ is hydrogen or a $C_1$ to $C_7$ alkyl group and $Y^1$ is hydrogen or a group shown by —O—Z or

$$-\underset{\underset{(O)_n}{\|}}{S}-Z,$$

(wherein Z is hydrogen, a $C_1$—$C_7$ alkyl group, a carboxy $C_1$—$C_7$ alkyl group, a $C_1$—$C_7$ alkoxycarbonyl group or an aryl-substituted $C_1$ to $C_7$ alkyl group and n is 0, 1, or 2), or a salt thereof, which comprises reacting a compound represented by formula IVa

$$R^1—N{=}C{=}Y \qquad \text{IVa}$$

wherein Y is oxygen or sulfur with a compound represented by formula Va

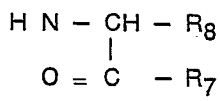
Va

and optionally further comprises the step of oxidising, alkylating or desulfurizing the reaction product.

3. A 3,5-di-tert-butyl-4-hydroxyphenyl-substituted heteocyclic compound represented by the following formula I, or a salt thereof:

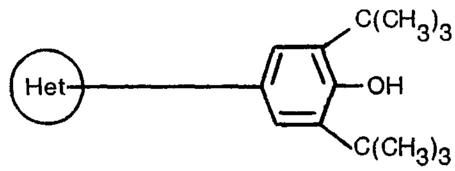

I

wherein

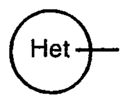

is a hetetocyclic group of the formula

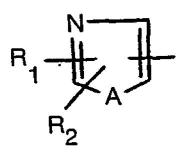

wherein $R_1$ and $R_2$ are the same or different and selected from hydrogen, $C_1$ to $C_7$ alkyl groups, aryl-substituted $C_1$ to $C_7$ alkyl groups, aryl groups, $C_1$ to $C_7$ alkoxy-substituted aryl groups, and the groups —O—Z

$$\underset{(O)_n,}{-S-Z,}$$

and —NH—Z (wherein Z represents hydrogen, a $C_1$ to $C_7$ alkyl group, a ($C_1$ to $C_7$ alkoxy) carbonyl $C_1$ to $C_7$ alkyl group, an aryl-substituted $C_1$ to $C_7$ alkyl group, or an aryl group and n is 0, 1, or 2); A is oxygen, sulfur, or >NR— where R is hydrogen or $C_1$ to $C_7$ alkyl or 3,5-di-tert-butyl-4-hydroxyphenacyl group.

4. 5-methyl- or 5-ethyl-4-(3,5-di-tert-butyl-4-hydroxyphenyl)-2-oxo-imidazoline.

5. 5-methyl- or 5-ethyl-4-(3,5-di-tert-butyl-4-hydroxyphenyl)-2-methylthioimidazole.

6. 5-methyl- or 5-ethyl-4-(3,5-di-tert-butyl-4-hydroxyphenyl)-imidazole.

7. 4-(3,5-di-tert-butyl-4-hydroxyphenyl)-2-mercaptothiazole.

8. 4-(3,5-di-tert-butyl-4-hydroxyphenyl)-2-methylthiothiazole.

9. A pharmaceutical composition containing a compound according to any one of claims 3 to 8 and an excipient.

**Patentansprüche**

1. Verfahren zur Herstellung einer 3,5-Di-tert-butyl-4-hydroxyphenyl-susbstituierten heterozyklischen Verbindung, die durch die nachstehende Formel Ia—1 wiedergegeben ist oder ein Salz derselben:

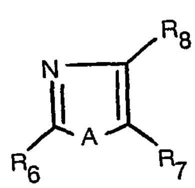

Ia—1

worin einer von $R_6$, $R_7$ und $R_8$ die 3,5-Di-tert-butyl-4-hydroxyphenylgruppe ist und die anderen gleich oder verschieden und ausgewählt aus Wasserstoff, $C_1$ bis $C_7$ Alkylgruppen, Aryl-substituierte $C_1$ bis $C_7$ Alkylgruppen, Arylgruppen, $C_1$ bis $C_7$ Alkoxysubstituierte Arylgruppen und den Gruppen —O—Z, —S—Z, und —NH—Z sind (worin Z

$(O)_n$

Wasserstoff, eine $C_1$ bis $C_7$ Alkylgruppe, eine Carboxy $C_1$ bis $C_7$ Alkylgruppe, eine ( $C_1$ bis $C_7$) Alkoxycarbonyl $C_1$ bis $C_7$ Alkylgruppe, eine Arylsubstituierte $C_1$ bis $C_7$ Alkylgruppe oder eine Arylgruppe

bedeutet und n 0, 1 oder 2 ist); A ist Sauerstoff, Schwefel oder >NR, worin R Wasserstoff oder eine $C_1$ bis $C_2$-alkyl-oder, 3,5-Di-tert-butyl-4-hydroxyphenacylgruppe ist, welches Verfahren umfaßt, das Umsetzen einer durch die Formel IIa wiedergegebenen Verbindung.

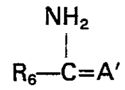

IIa,

worin A' Sauerstoff, Schwefel, eine Iminogruppe oder eine $C_1$ bis $C_7$ Alkyliminogruppe ist und $R_6$ wie oben definiert ist, mit einer durch die Formel IIIa wiedergegebenen Halogenocarbonylverbindung

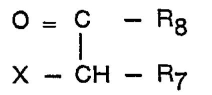

IIIa,

worin X Halogen und $R_7$ und $R_8$ wie oben definiert sind, und gewünschtenfalls dann das Oxidieren, Akylieren oder Desulferieren des erhaltenen Produktes.

2. Ein Verfahren zum Herstellen einer 3,5-Di-tert-butyl-4-hydroxyphenyl-susbstituierten heterozyklischen Verbindung, welche durch die nachstehende Formal Ia—2 wiedergegeben ist, oder ein Salz derselben:

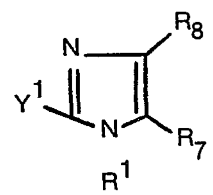

Ia—2

worin einer von $R_7$ oder $R_8$ die 3,5-Di-tert-butyl-4-hydroxyphenylgruppe ist und der andere gleich oder verschieden und ausgewählt ist aus Wasserstoff, $C_1$ bis $C_7$ Alkylgruppen, Aryl-substituierte $C_1$ bis $C_7$ Alkylgruppen, Arylgruppen, $C_1$ bis $C_7$ Alkoxy-substuierte Arylgruppen, und den Gruppen —O—Z,

$$-\underset{(O)_n}{S}-Z,$$

und —NH—Z (worin Z Wasserstoff, eine $C_1$ bis $C_7$ Alkylgruppe, eine Carboxy $C_1$ bis $C_7$ Alkylgruppe ($C_1$ bis $C_7$ Alkoxy)carbonyl $C_1$ bis $C_7$ Alkylgruppe, eine Aryl-substituierte $C_1$ bis $C_7$ Alkylgruppe oder eine Arylgruppe bedeutet und n 0, 1 oder 2 ist); $R^1$ Wasserstoff oder eine $C_1$ bis $C_7$ Alkylgruppe und $Y^1$ Wasserstoff oder eine durch O—Z — oder

$$-\underset{(O)_n}{S}-Z-$$

widergegebene Gruppe ist, (worin Z Wasserstoff, eine $C_1$ bis $C_7$ Alkylgruppe, eine Carboxy $C_1$ bis $C_7$ Alkylgruppe, eine $C_1$ bis $C_7$ Alkoxycarbonylgruppe oder eine Aryl-substituierte $C_1$ bis $C_7$ Alkylgruppe ist und n 0, 1 oder 2 ist), oder ein Salz derselben, welches Verfahren umfaßt, das Umsetzen einer durch die Formel IVa wiedergegebenen Verbindung

$$R^1—N=C=Y \qquad Va,$$

worin Y Sauerstoff oder Schwefel ist, mit einer durch die Formel Va wiedergegebenen Verbindung

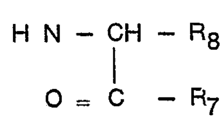

Va,

und gegebenenfalls weiters umfaßt die Stufen des Oxidierens, Alkylierens oder Desulfurierens des Reaktionsproduktes.

3. Eine 3,5-Di-tert-butyl-4-hydroxyphenyl-substituierte heterozyklische Verbindung, welche durch die folgende Formel I wiedergegeben ist oder ein Salz derselben:

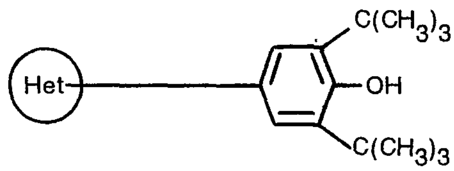

I

worin

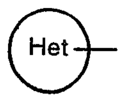

eine heterozyklische Gruppe der Formel

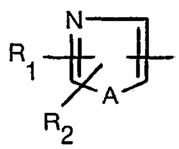

ist, worin $R_1$ und $R_2$ gleich oder verschieden sind und ausgewählt sind aus Wasserstoff, $C_1$ bis $C_7$ Alkylgruppen, Aryl-susbstituierten $C_1$ bis $C_7$ Alkylgruppen, Arylgruppen, $C_1$ bis $C_7$ Alkoxy-substituierte Arylgruppen und aus den Gruppen —O—Z,

$$\underset{(O)_n}{—S—Z}$$

und —NH—Z (worin Z Wasserstoff, eine $C_1$ bis $C_7$ Alkylgruppe, eine Carboxy $C_1$ bis $C_7$ Alkylgruppe, eine ( $C_1$ bis $C_7$ Alkoxy)carbonyl $C_1$ bis $C_7$ Alkylgruppe, eine Aryl-substituierte $C_1$ bis $C_7$ Alkylgruppe, oder eine Arylgruppe bedeutet und n 0, 1 oder 2 ist); A ist Sauerstoff, Schwefel oder >NR, worin R Wasserstoff oder $C_1$ bis $C_7$ Alkyl-oder 3,5-Di-tert-butyl-4-hydroxyphenyacylgruppe ist.

4. 5-Methyl- oder 5-Äthyl-4-(3,5-di-tert-butyl-4-hydroxyphenyl)-2-oxo-imidazolin.

5. 5-Methyl- oder 5-Äthyl-4-(3,5-di-tert-butyl-4-hydroxyphenyl)-2-methylthioimidazol.

6. 5-Methyl- oder 5-Äthyl-4-(3,5-di-tert-butyl-4-hydroxyphenyl)-imidazol.

7. 4-(3,5-Di-tert-butyl-4-hydroxyphenyl)-2-mercaptothiazol.

8. 4-(3,5-Di-tert-butyl-4-hydroxyphenyl)-2-methylthiazol.

9. Eine pharmazeutische zusammensetzung enthaltend eine verbindung nach einem der anspruche 3 bis 8 und ein hilfsmittel.

**Revendications**

1. Procédé de préparation d'un composé hétérocyclique substitué par le groupement di-tert-butyl-3,5-hydroxy-4-phényle et représenté par la formule Ia—1 ci-après ou d'un sel de celui-ci:

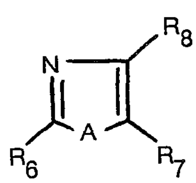

Ia—1

dans laquelle l'un de $R_6$, $R_7$ et $R_8$ est le groupe di-tert-butyl-3,5-hydroxy-4-phényle et les autres sont semblables ou différents et choisis entre l'hydrogène, les groupes alkyle en $C_1$ à $C_7$, les groupes alkyle en $C_1$ à $C_7$ à substituant aryle, les groupes aryle, les groupes aryle à substituant alcoxyle en $C_1$ à $C_7$ et les groupes —O—Z,

$$\underset{(O)_n}{—S—Z}$$

et —NH—Z (dans lesquels Z représente un hydrogène, un groupe alkyle en $C_1$ à $C_7$, un groupe carboxy-(alkyle en $C_1$ à $C_7$), un groupe (alcoxy en $C_1$ à $C_7$)-carbonyl-(alkyle en $C_1$ à $C_7$), un groupe alkyle en $C_1$ à $C_7$ a substituant aryle, ou un groupe aryle, et *n* est 0, 1 ou 2); A est un oxygène, un soufre ou >NR dans lequel R

est un hydrogène ou un groupe alkyle en $C_1$ à $C_7$ ou un groupe di-tert-butyl-3,5-hydroxy-4-phénacyle, qui consiste à faire réagir un composé représenté par la formule IIa:

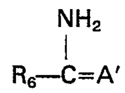

(IIa)

dans laquelle A' est un oxygène, un soufre, un groupe imino ou un groupe alkylimino en $C_1$ à $C_7$ et $R_6$ est tel que défini ci-dessus, sur un composé halogénocarbonylé représenté par la formule IIIa:

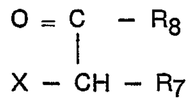

(IIIa)

dans laquelle X est un halogène et $R_7$ et $R_8$ sont tels que définis ci-dessus, et ensuite, si on le désire, à oxyder, à alkyler ou à désulfurer le produit obtenu.

2. Procédé de préparatio d'un composé hétérocyclique substitué par le groupement di-tert-butyl-3,5-hydroxy-4-phényle représenté par la formule Ia—2 ci-après ou d'un sel de celui-ci:

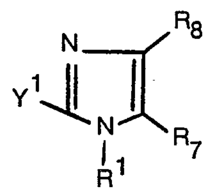

Ia—2

dans lequelle l'un de $R_7$ et $R_8$ est le groupe di-tert-butyl-3,5-hydroxy-4-phényle et l'autre est semblable ou différent et choisi entre l'hydrogène, les groupes alkyle en $C_1$ à $C_7$, les groupes alkyle en $C_1$ à $C_7$ à substituant aryle, les groupes aryle, les groupes aryle à substituant alcoxyle en $C_1$ à $C_7$ et les groupes —O—Z,

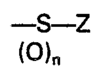

et —NH—Z (dans lesquels Z représente un hydrogène, un groupe alkyle en $C_1$ à $C_7$, un groupe carboxy-(alkyle en $C_1$ à $C_7$), un groupe (alcoxyle en $C_1$ à $C_7$)-carbonyl-(alkyle en $C_1$ à $C_7$), un groupe alkyle en $C_1$ à $C_7$ à substituant aryle, ou un groupe aryle, et *n* est 0.1 ou 2); $R^1$ est un hydrogène ou un groupe alkyle en $C_1$ à $C_7$ et $Y^1$ est un hydrogène ou un groupe représenté par —O—Z ou

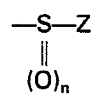

(dans lequel Z est un hydrogène, un groupe alkyle en $C_1$ à $C_7$, un groupe carboxy-(alkyle en $C_1$ à $C_7$). un groupe (alcoxy en $C_1$ à $C_7$)-carbonyle ou un groupe alkyle en $C_1$ à $C_7$ à substituant aryle et *n* est 0, 1 ou 2), ou d'un sel de celui-ci, qui consiste à faire réagir un composé représenté par la formule IVa:

$R^1$—N=C=Y (IVa)

dans laquelle Y est un oxygène ou un soufre, sur un composé représenté par la formula Va:

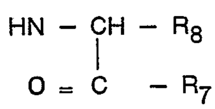

(Va)

et comprend facultativement en outre l'étape consistant à oxyder, à alkyler ou à désulfurer le produit de la réaction.

3. Composé hétérocyclique substitué par le groupement di-tert-butyl-3,5-hydroxy-4-phényle, représenté par la formule I ci-après, ou sel de celui-ci:

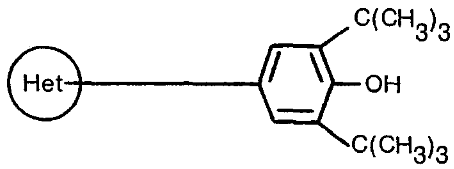

I

dans laquelle

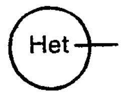

est un groupe hétérocyclique de formule:

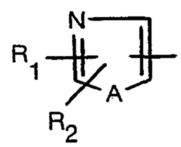

dans laquelle $R_1$ et $R_2$ sont semblables ou différents et choisis entre l'hydrogène, les groupes alkyle en $C_1$ à $C_7$, les groupes alkyle en $C_1$ à $C_7$ à substituant aryle, les groupes aryle, les groupes aryle à substituant alcoxyle en $C_1$ à $C_7$, et les groupes —O—Z,

$$-\underset{(O)_n}{S}-Z$$

et —NH—Z (dans lequels Z représente un hydrogène, un groupe alkyle en $C_1$ à $C_7$, un groupe carboxy-(alkyle en $C_1$ à $C_7$), un groupe (alcoxy en $C_1$ à $C_7$)-carbonyl-(alkyle en $C_1$ à $C_7$), un groupe alkyle en $C_1$ à $C_7$ à substituant aryle, ou un groupe aryle et *n* est 0, 1 ou 2); A est un oxygène, un soufre ou >NR dans lequel R est hydrogène ou un groupe alkyle en $C_1$ à $C_7$ ou un groupe di-tert-butyl-3,5-hydroxy-4-phénacyle.

4. Méthyl-5- ou éthyl-5-(di-tert-butyl-3,5-hydroxy-4)-phényl-4-oxo-2-imidazoline.

5. Méthyl-5- ou éthyl-5-(di-tert-butyl-3,5-hydroxy-4)-phényl-4-methyl-2-thioimidazole.

6. Méthyl-5- ou éthyl-5-(di-tert-butyl-3,5-hydroxy-4)-phényl-4-imidazole.

7. (di-tert-butyl-3,5-hydroxy-4)-phényl-4-mercapto-2-thiazole.

8. (di-tert-butyl-3,5-hydroxy-4)-phényl-4-méthyl-2-thiothiazole.

9. Une composition pharmaceutique contenant un compose selon, l'une quelconque des revendications 3 a 8 en un excipient.